# EUROPEAN PATENT SPECIFICATION

(11) **EP 0 880 338 B1**
(45) Date of publication and mention of the grant of the patent: **26.10.2005**
(21) Application number: 97905873.2
(22) Date of filing: 13.02.1997
(51) Int. Cl.: A61B 1/32, A61B 1/00, A61M 25/00, A61M 29/00, A61M 16/00

(54) **SURGICAL ACCESS DEVICE**
CHIRURGISCHE ZUGANGSVORRICHTUNG
DISPOSITIF D'ACCES CHIRURGICAL

(30) Priority: 13.02.1996 US 600603; 13.02.1996 US 600428; 13.02.1996 US 600422; 13.02.1996 US 600608
(43) Date of publication of application: 02.12.1998
(73) Proprietor: CONMED CORPORATION, Utica, NY 13502 (US)
(72) Inventor: BACICH, Steven, R., Halfmoon Bay, CA 94019 (US); GREELIS, John, P., Aliso Viejo, CA 92656 (US); NGUYEN, Hien, Santa Ana, CA 92704 (US); NGUYEN, Tuoc, Westminster, CA 92683 (US)
(74) Representative: Popp, Eugen
(86) International application number: PCT/US1997/002056
(87) International publication number: WO 1997/029680

(56) References cited:
- WO-A-95/18562
- US-A- 3 789 852
- US-A- 4 211 234
- US-A- 4 406 656
- US-A- 5 025 778
- US-A- 5 213 092
- US-A- 5 217 001

## Description

### Field of the Invention

The present invention relates to a surgical access device comprising: an elongate tubular element with an open distal end, providing a first channel. At least one secondary channel giving an auxiliary lumen for the insertion of an instrument, endoscope or other visualisation device being further provided.

### Background of the Invention

The advantages of minimally invasive surgery are well known and understood. Essentially, through the use of advanced endoscopy and other vision systems, surgery can be performed percutaneously through one or more small incisions or portals formed in the patient's body or through a bodily orifice, such as vagina, cervix, urethra, rectum, mouth, etc. Entrance to the body is accomplished in a number of ways depending upon the type of procedure. Once a partal or "port" is formed in the patient's body, a number of surgical access devices may be placed therethrough in order to perform the endoscopic procedure. Such devices will typically include some form of endoscope or other vision system to allow the surgeon to visualize the procedure. However, other surgical access devices may be used in combination with an endoscope, such as an introducer, an endoscopic sheath, catheters, or other cannulas. Thus, endoscopes and other endoscopic surgical instruments may be inserted through these surgical access devices which may have one or more instrument channels formed therein. Such surgical access devices may be reusable and, thus, require sterilization (such as most endoscopes), or may be disposable (such as introducers, endoscopic sheaths, etc.).

Minimaliy invasive surgery obviousiy reduces the trauma and pain to the patient, accelerates recovery, and shortens the average hospital stay, thus minimizing the costs of health care in the U.S. and around the world. In addition to minimal invasiveness, there is also a trend to attempt to perform unanticipated procedures during the initial surgery so as to avoid scheduling repetitive surgeries. That is, for example, frequently a diagnostic procedure is scheduled for a given purpose; however, once inside the patient, the surgeon notices a cyst, polyp, lesion, or other suspicious pathology. Therefore, the surgeon may desire to perform a biopsy or other surgical procedure. If an additional diagnostic or therapeutic procedure could be accomplished concurrently with the initial procedure, substantial savings in patient comfort, recovery time, and costs could be realized. However, presently in most cases, the patient must be rescheduled for a later procedure.

Although it is known in the prior art to provide auxiliary, expandable channels in surgical access devices, they have apparently not met with commercial success. A number of reasons may be postulated.

Although it is understood that the surgical access device must initially have a small cross-sectional profile for ease of insertion, the means for expanding that device have varied. Typically, such expansion means comprises a secondary or auxiliary channel having a lumen for the insertion of an andoscope or other endoscopic instrument. Thus, the main lumen of the surgical access device is formed by a hollow channel defined by a certain wall of thickness. The cross-sectional profile of the surgical access device is usually circular, although other profiles have been utilized. As used herein, "profile" will mean a cross-sectional profile unless otherwise specified. Thus, the goal of present access devices is to minimize their profits upon initial introduction. Following insertion, however, it is desirable to form a secondary channel in the device for the insertion of a second instrument in order to complete the intended procedure or another unanticipated procedure. This secondary channel is typically formed from a polymeric or rubberized elastic material. Due to their elastic nature, such secondary channels have substantial wall thicknesses. Moreover, in order to minimize the profile of the device upon insertion, these secondary channels must be collapsed in some fashion upon insertion. Thus, the cross-sectional wall thickness of the secondary channel must lie upon the outer diameter of the main channel, thus adding significantly to the overall profile of previous surgical access devices. This construction adds a new problem to the one the device is attempting to solve.

The most commonly proposed solution to the extra profile added by the secondary channel, is to surround it with an outer sheath or other elastic band, in order to hold it in a collapsed state around the outer diameter of the main channel of the surgical access device.

For example, U.S. Patent No. 5,025,778 discloses an endoscope having a flexible tubing adjacent the endoscope. The flexible tubing is held against an insertion tube by a sheath. The sheath is sufficiently flexible to permit the tubing to expand to the desired cross-sectional area. WO 95/18562 discloses a channel system for use with a conventional endoscope. The system has a collapsible access channel that is connected to a distal end of the endoscope. The endoscope may be provided with a protective sheath and/or an end cap that is integral with the collapsible channel.

However, this approach simply aggravates the problem due to the wall thickness of the outer sheath or banding. Moreover, these outer materials add to the radial resistance which must be overcome in order to push the instrument through the secondary channeL In addition, and quite significantly, the elastic nature of previous secondary channels presents severe frictional disadvantages, further intensifying the problem of instrument insertion. Moreover, in reusable systems, the outer sheathing or banding, which causes a secondary lumen to collapse, presents a substantial problem with respect to sterilization.

Another significant disadvantage of prior secondary channels is that they are elastically expandable, both longitudinally and radially. Thus, upon either insertion and/or deployment of secondary instrument, the channels may become loose or gathered. Thus, upon insertion of the instrument, there might be bunching or binding, which prevents the instrument from smoothly accessing its desired location. This requires the application of greater force on the instrument, thus increasing the pain and trauma to the patient which is intended to be avoided by the surgical access device. That is, most procedures of this type are performed on an out-patient basis with the patient undergoing only a local anesthetic. Thus, the difficulties associated with previous secondary channels, including their more frictional nature, increases the likelihood that the procedure will be uncomfortable and even traumatic for the patient.

Moreover, because of the elastic nature of previous secondary channels, they require an additional hollow tube to hold them in the open position for repetitive instrument insertion. Furthermore, there has been a lack of attention to leading edge design, so as to avoid contamination upon insertion of prior art surgical access devices. This is particularly a severe problem in connection with the usable systems which require sterilization between use.

Thus, there are substantial problems associated with secondary channels formed in a prior art surgical access devices. Moreover, previous devices have not addressed advances in endoscopic design. That is, initially endoscopes were of the straight and rigid rod lens type, or the more costly flexible scopes with articulating distal ends. However, more recent semi-rigid endoscopes are smaller in diameter and allow some flexibility in use, unlike rigid scopes. Semi-rigid endoscopes present new obstacles, but also additional opportunities, with respect to auxiliary channels, which opportunities have not been addressed by previous surgical access devices.

Accordingly, there is a severe need in the prior art for surgical access devices and methods for constructing them which can successfully provide secondary or auxiliary surgical channels.

### Summary of the Invention

The present invention satisfies this need in the prior art by providing a surgical access device according to claim 1, with preferred embodiments of the invention defined in the dependent claims. The surgical access device comprises:
an elongate tubular element with an open distal end, providing a first channel.

At least one secondary channel giving an auxiliary lumen for the insertion of an instrument, endoscope, or other visualisation device is provided.

The secondary channel is constructed from a substantially inelastic flexible membrane, which has a pre-release storage position and a dilated position. The membrane is dilated in use so as to allow the passage of the instrument, endoscope or other visualisation device.

Preferred embodiments and design features of the proposed surgical access device are referred to in the subsidiary claims.

The present invention further satisfies the need in the prior art by providing a surgical access device and method of constructing same wherein the secondary or auxiliary lumen comprises a guide channel formed from an extremely thin, but very strong and substantially noncompliant membrane. This guide membrane exhibits performance characteristics which make it preferred for this application.

The wall thickness of the membrane is so thin (approximately .001 inches in some embodiments) that it has only a negligible affect on the profile of the present surgical access device. This is true even though the membrane may be pleated, folded, or doubled back on or around the outer wall surface of the surgical access device. Thus, the guide membrane of the present invention is compatible with very small diameter access devices which are more commonty being used with rigid and especially semi-rigid endoscopes. Accordingly, the surgical access device of the present invention is able to substantially reduce the pain and trauma associated with endoscopic procedures.

One important advantage of the present membrane is that it can be formed or set in position on the surgical access device. That is, by the use of heat-forming or heat-shrinking techniques, or other mechanical or chemical (e.g., adhesives) means, the membrane can be "set" in order to closely conform to the outer surface configuration of the access device, thus maintaining a narrow or otherwise small profile. Moreover, outer elastic sheathing, straps, or binding of any type are unnecessary; thus, the profile of the access device is further minimized. An additional advantage of the present membrane is its lubricity. That is; the material in its natural state as formed on the access device is lubricous or otherwise less-adhesive, thereby facilitating inserting of the access device and reducing discomfort.

The guide channel membrane of the present invention can be constructed from any one of a number of highly oriented or cross-linked, noncompliant materials, including, without limitation, polymers. Such polymers may preferably undergo an extrusion process in order to achieve their high orientation status, resulting in their noncompliant and substantially inelastic nature. Moreover, such extruded polymers are also very strong and tough, and lubricous as pointed out above. In the preferred embodiment, one guide channel membrane material is polyethylene terephthalate ("PET"); although other materials within that group are possible, examples being polyolefins and their blends which can be highly orientated or cross-linked after radiation treatment and heat forming as found in the art of balloons for angioplasty catheters. Other material include nylon and polyethylene which achieve orientation by pre-stretching whereby the material has high strength and little elongation when a load (stress) is exerted upon it.

The guide channel membrane may be formed from material having various thicknesses, depending upon the application of the particular surgical access device; however, thicknesses in the range of .0005-.002 inches are preferred. Thus, it can be seen that such membranes do not add significantly to the profile of the access device.

Another advantage of the guide channel membrane of the present invention is that they are "releasable" upon dilation. That is, although heat formed or otherwise set so as to closely conform to the outer configuration of the access device, the membrane material can easily open up or release to form a secondary guide channel. In most cases, dilation can be achieved by the secondary endoscopic instrument itself, without a need for a dilator or obturator. Thus, these additional steps can be avoided. Moreover, the materials are also internally lubricous, thus, minimizing resistance to instrument insertion and advancement. The lubricous nature can also eliminate the need for additional layers of material, such as Teflon and their coatings, which can add profile as well as cost to the device. Since the membrane material is not elastic and is otherwise releasable, there is no radial resistance to instrument advancement. In addition, no internal support is necessary. That is, once the membrane material has been released, it forms a secondary channel which conforms to the nature of the tissue around it. In other words, if the tissue surrounding the access device and secondary channel is tight, the membrane will coliapse and conform at the tissue in order to avoid unnecessary trauma. On the other hand, if the passage is expanded or dilated, the channel, following release, will maintain its general channel-like shape, without the need for any auxiliary internal tubing or support from any media such as fluid. Thus, the membrane will maintain its configuration even with the instrument removed.

The guide channel of the present invention is self-adjusting. That is, the membrane material will release to form a secondary channel which is only large enough to admit the passage of the instrument being advanced through it. Thus, the guide channel holds the instrument securely along its path as it is advanced to the distal end of the access device. This advantage also allows for insertion of instruments having various cross-sectional profiles, thus avoiding the need to design secondary channels specifically for certain instruments. In certain embodiments, perforations or slits may be formed in the guide channel in order to facilitate release or dilation.

As noted above, the guide channel membrane is distensible, but substantially noncompliant. Thus, it will not expand elastically upon insertion or dilation, either longitudinally or radially. It will be understood that the term "radially" is intended to mean in an outward direction, the cross-sectional configuration of the present guide channel not being limited to a circular or cylindrical configuration. Thus, the guide channel will not bunch up or bind as the instrument is advanced through it. Moreover, because of its toughness and strength, repetitive insertions of the instrument without failure are readily achievable, especially in tight or strong tissue, such as experienced in laparoscopic applications. In addition, the membrane under these conditions will not experience longitudinal expansion, which could result in the guide channel extending beyond the distal end of the introducer, thereby blocking or obscuring vision of the endoscope.

Upon withdrawal, the guide channel membrane is easily collapsible so as to minimize any pain or trauma. Moreover, with the application of a slight vacuum, the membrane will conform closely to the outer surface configuration of the surgical access device for easy withdrawal.

The surgical access device of the present invention can be constructed from inexpensive materials and in accordance with simple construction techniques. This is particularly true of the guide channel membrane. Thus, the access device is disposable, thus avoiding problems associated with sterilization. Moreover, the membrane is compatible with any type of surgical access device, including introducers, endoscopic sheaths, catheters, cannulas, and endoscopes themselves.

In accordance with another advantage of the present invention, one embodiment of the membrane described above is used to form a guide channel on the surgical access device. Unlike secondary channels of the prior art, the present guide channel can be used to guide an instrument through a bend or curve as may be experienced in a procedure using a semi-rigid endoscope. In other words, such endoscopes are often used in connection with curved introducers which allow them to navigate certain curved anatomical paths and/or to move or visualize tissue. Thus, in accordance with one aspect of the present invention, the guide channel is nonlinear. Such a channel can be formed upon a guide platform which is formed on or is otherwise associated with the access device. Moreover, guide rails can be formed to further provide structure and rigidity to the secondary channel. The bends or curves formed in the guide channel to orient the secondary lumen so that the instrument can arrive at a specific distal location with respect to the primary lumen, depending upon the procedure.

The guide platforms or rails can take on a number of configurations. Advantageously, however, due to the formable and thermoplastically settable nature of the channel membrane, the channel membrane can be folded or arranged with respect to the access device in a wide variety of ways.

The surgical access device of the present invention also exhibits a particular distal end design which avoids contamination. Upon insertion of the device, the instrument channel is sealed so as to avoid entry of tissue or foreign contaminating material. Due to the thermoplastic nature of the channel membrane, the seal can be accomplished by heat forming the channel at the distal tip. Alternatively, a narrow profile tip can be designed which plugs the distal opening of the secondary channel while still facilitating entry of the access device. Like the distal end of the present access device, the proximal end also features a particular "y" design which facilitates advancement of a secondary instrument into the guide channel while minimizing risk of damage to the device or discomfort to the patient. The proximal end of the access device is provided with a housing which gently introduces the instrument along a path which eventually becomes tangential to the main longitudinal axis of the access device. The housing which surrounds the proximal end is also provided with appropriate valves to control and regulate the in-flow and out-flow of distension media, irrigation fluid, or other fluid.

As noted above, the guide channel of the present invention can be integrally formed on the insertion tube of an endoscope or separately formed on an introducer, endoscopic sheath, and the like. In the latter case, the introducer can be designed and constructed so as to guide the entrance of the secondary instrument in a particular way in order to achieve a specific purpose, depending upon the procedure being accomplished. Moreover, the instrument can enjoy a lumen independent of any movement of the endoscope which is inserted through the main channel of the introducer.

In accordance with the method of construction of the surgical access device of the present invention, as noted above, the settable nature of the membrane material facilitates a number of construction arrangements and techniques. Thus, the membrane material can be folded or stored with respect to the access device on an exterior surface, interior surface, or other intermediate location. It can be coupled to the de vce by a wide variety of means, including mechanical, adhesive, heat formation, etc.

Thus, in accordance with a preferred method, the surgical access device of the present invention is constructed from a main tube which provides a main channel for the access device. The main tube can be constructed from stainless steel tubing or a rigid plastic such as polycarbonate which can provide strength with little wall thickness. Typically, the main channel provides access for insertion or introduction of an endoscope; however, other instruments can be introduced into the patient as well through the main channel. The guide channel membrane is formed onto the main tube in the following manner. The membrane is provided in the form of a hollow tube, which is typically extruded to form that shape, so as to have an outer diameter which is greater than that of the main tube. The membranes can be constructed from PET tubing which can come in the form of balloon tubing which is pre-stretched and highly orientated for minimal elongation. Other constructions of membranes can use polyolefins and their blends, polyethylene, and nylons which are highly orientated or cross-linked. The guide channel membrane tube is placed over the main channel tube and positioned eccentrically with respect to the axis thereof.

A split tube sheath is mechanically clamped over the main tube capturing the guide channel membrane tube against the main tube. The split tube can be made from nylon 11 which has high strength with little wall thickness. Other materials such as polycarbonate, polyethylene, urethane, and the like can be employed. The split channel can be mechanically affixed to the main tube or be placed onto the main tube by a variety of adhesive agents or thermal bonding techniques. The actual width of the slit itself can vary which will affect the profile and guiding characteristics of the membrane channel. The excess membrane material, owing to the fact that its outer diameter is greater than that of the main tube, is allowed to escape through the slit in the sheath and extends outwardly therefrom. This excess material is then folded, pleated, or otherwise stored with respect to the main tube in any one of a variety of ways so as to minimize the profile of the surgical access device. Typically, the excess membrane material is folded or doubled back on itself so as to closely conform to the outer surface configuration of the tube. An intermediate amount of heat, such as approximately 71.1°C (160°F), is then applied to the membrane material so as to heat form or set it in position closely conforming to the main tube, although other mechanical forming or adhesive techniques may be employed. The settable nature of the membrane is such that a crease or seam formed in the pleated material will retain a sharp, narrow profile, thus facilitating entry and use and avoiding damage or distortion to the guide channel under these conditions.

In accordance with another method of construction and introducer embodiment, an even narrower profile introducer can be constructed without the need for an outer split sheath. In this case the guide channel membrane tube is heat bonded or otherwise coupled directly to the hypotube by adhesive or other means. To facilitate this construction, the membrane tube can be supplied in a multi-lumen or figure-8 configuration, wherein the membrane is constructed from an extrusion or other process. Moreover, one or more of the lumens may be collapsible, and the others may be noncellapsible, either due to their increased wall thickness or to rigidifying means such as hypotubes or reinforcement devices, etc.

In accordance with another step of the present method, a merge channel may be formed along the main channel in order to provide for the easy insertion of a secondary instrument into the guide channel. The merge channel can be constructed from a variety of materials including nylon 11 and other polymers as well as stainless steel which can be flexed yet retain radial integrity. As noted above, the merge channel is proximally located with respect to the surgical access device and remains substantially out of the body. In accordance with the present method, the merge channel tube is longitudinally aligned with respect to the axis of the main tube prior to the overwrapping of the guide channel membrane tube. Thus, the proximal end of the membrane tube circumscribes both the main tube and the distal end of the merge channel tube and the guide channel is simultaneously formed around both the main tube and the merge channel tube to comprise the "y" junction of the surgical access device. To provide mechanical strength at this "y" junction, a housing or other mechanical clamping means is provided. The housing can take a variety of forms to provide ergonomic benefits to the operator or clinician. in construction, it can be made from a variety of injection molded plastics including polycarbonate, polysulfone, nylon, etc.; or machined. It can be a part of the disposable introducer or a separate unit which is reusable, re-sterilized, and placed back onto the surgical access device by the operator prior to each use.

In another step of the present invention, the distal tip of the surgical device is sealed so as to prevent contamination or distortion of the guide channel upon insertion of the access device into the body. Likewise, at the proximal end of the access device, the main channel and merge channel are provided with the necessary valving for irrigation or distention media. The valving must prevent any leakage around an instrument or endoscope when these devices are placed through the ports and typically has an 0-ring or washer type structure. In addition, they must contain structures such as duck-bill or star valves which prevent the back flow of media through the ports when no instrument or endoscope is through the port. These valves can be made from silicone, rubber, and other elastomeric materials which are known in the art.

Thus, in summary, the surgical access device of the present invention comprises a first channel having a first lumen for the insertion of an instrument, endoscope, or other visualization device, and at least one secondary channel for providing an auxiliary lumen for the insertion of an instrument, endoscope, or other visualization device, wherein the secondary channel is mounted along the outer surface of the surgical access device and is constructed from a thin pleated membrane which, prior to dilation of said secondary channel, so closely conforms to the outer surface of the device so as to only negligibly increase the size of the profile of the device. The present invention further comprises a surgical access device as described above, wherein the secondary channel is constructed from a membrane which is self-conformable to the device and which is settable, so as to closely conform to the surgical access device.

Accordingly, a surgical access device of the present invention and the method of constructing it provide a substantial advancement over the prior art.

### Brief Description of the Drawings

FIGURE 1 is a side view of the surgical access device of the present invention illustrating a partially inserted endoscope and a secondary instrument in a pre-introduction location.
FIGURE 2 is a close-up side view of the access device illustrating the advancement of the secondary instrument through the guide channel of the present invention.
FIGURE 3 is a cross-sectional view of the access device taken along line 3-3 of FIGURE 2 illustrating the main channel and guide channel in their initial state upon insertion of the access device into the body and prior to deployment of a secondary instrument through the guide channel.
FIGURE 3a is a cross-sectional view of the access device illustrating an alternative embodiment of the main channel and guide channel prior to deployment of a secondary instrument through the guide channel.
FIGURE 3b is a cross-sectional view of the present access device illustrating an alternative embodiment for the guide channel which does not utilize a split sheath.
FIGURE 3c is a cross-sectional view of the access device of FIGURE 3b, illustrating the guide channel in its deployed or released position.
FIGURE 4 is a cross-sectional view of the access device taken along lines 4-4 of FIGURE 2 illustrating the guide channel of the present invention in a released or expanded state as the secondary instrument is advanced therethrough.
FIGURE 5 is a graph illustrating the stress-strain relationship of oriented PET, as used for the guide channel membrane, in comparison with a typical elastomer.
FIGURE 6 is a graph illustrating the relationships between tube diameter and internal pressure for tubes comprising PVC, polyolefins, and PET material.
FIGURE 7 is a close-up perspective view of the distal tip of the present access device illustrating a bend or nonlinear curve which may be formed in the device, and further illustrating the manner in which the present guide channel allows a secondary instrument to conform to such curve.
FIGURE 8 is a close-up, broken-away view of the access device of FIGURE 6 illustrating a guide platform or guide rail formed in conjunction with guide channel which guides and supports the secondary instrument along the curved guide channeL
FIGURE 9 is a cross-sectional view of another embodiment of the surgical access device of the present invention illustrating other guide channel and guide platform configurations, together with the manner in which the guide channel membrane is formed with respect thereto.
FIGURE 10 is another embodiment of the guide channel of the present invention illustrating a guide channel membrane which is perforated or otherwise slit in order to exhibit unique or specialized release characteristics.
FIGURE 10a is the guide channel embodiment of FIGURE 10 with an instrument deployed substantially along the length of the guide channel.
FIGURE 11 is a close-up perspective view of the distal tip of the present access device illustrating the manner in which the guide channel may be sealed to prevent contamination or damage to the guide channel upon introduction of the access device into the body.
FIGURE 11a is a close-up perspective view of an alternative embodiment of the distal tip of the present access device illustrating another manner in which the guide channel may be sealed to prevent contamination or damage to the guide channel upon introduction of the access device into the body.
FIGURE 12 is a longitudinal cross-sectional view taken through the proximal housing of the present access device in order to illustrate the merge channel leading into the guide channel of the present invention.
FIGURE 13 is a cross-sectional view taken along lines 13-13 of FIGURE 12 illustrating the merge channel and main channel distal the proximal housing of the present access device.
FIGURE 14 is an exploded view of the present access device illustrating the method of its construction.
FIGURE 15 is an elevational view along the longitudinal axis of the distal end of another embodiment of the present invention comprising an introducer having an open-loop formed thereon.
FIGURE 16 is a cross-sectional detail view of the open-loop of FIGURE 15 showing a hole formed therethrough.
FIGURE 17 is a cross-sectional view showing the open-loop introducer of FIGURE 15 with an aspiration tube positioned in the guide channel.
FIGURES 18 and 18a are cross-sectional views of another embodiment of FIGURES 3 and 3a, illustrating the main or laparoscopic channel of the access device and a secondary guide channel which is shown in its initial storage or collapsed position with respect to the access device body and prior to the deployment of a secondary instrument through said guide channel.
FIGURE 19 is a cross-sectional view of the embodiment of the present access device shown in FIGURES 18 and 18a, as taken along lines 4-4 of FIGURE 2, illustrating the guide channel of the present invention in a released or distended state as a secondary laparoscopic instrument is advanced therethrough.
FIGURE 20 is a cross-sectional view of the laparoscopic embodiment of the access device of the present invention illustrating an alternate embodiment of the guide channel.
FIGURE 21 is a cross-sectional view of the access device of FIGURE 20 illustrating the insertion of a secondary instrument along the guide channel.
FIGURE 22 is a close-up side view of an alternate distal tip of the present access device.
FIGURE 23 is a close-up view of the distal tip of another embodiment of the surgical access device illustrating the secondary instrument and the uterine tissue being visualized by a hysteroscope.

### Detailed Description of the Preferred Embodiment

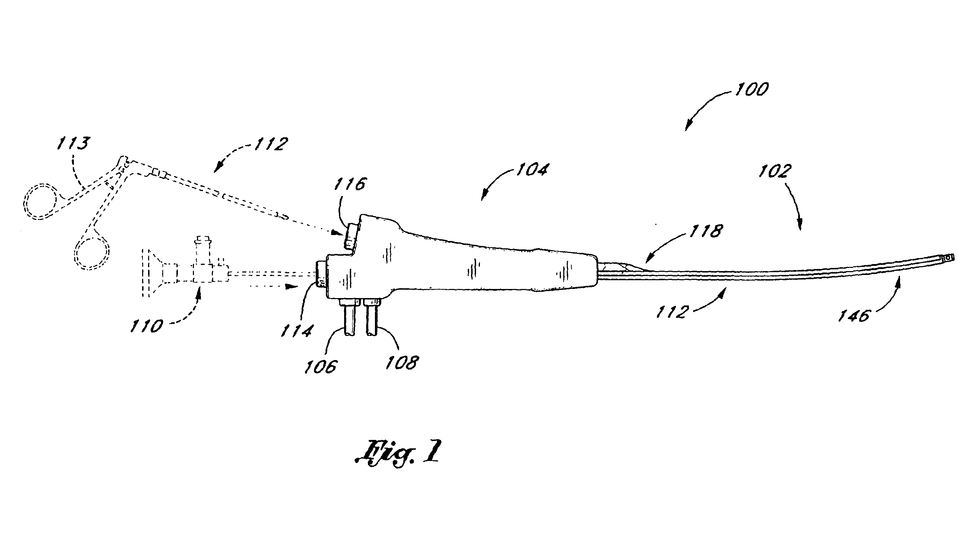

With reference to FIGURE 1, there is shown the surgical access device 100 of the present invention. In this case, a surgical introducer has been selected to illustrate the principles of the present invention; however, it will be understood that such principles apply equally to all types of surgical access devices, as well as to devices not necessarily limited to surgical access. In the broadest sense, the principles of the present invention encompass devices where secondary channels or other types of guide channels, expandable or otherwise, are desirable or necessary in order to allow passage of some type of instrument. Such devices include, without limitation, introducers, endoscopic sheaths, catheters, cannulas, and the like. The secondary or guide channels of the present invention may be retro-fitted onto such devices or integrally formed therein. For example, the guide channel of the present invention may be integrated into the insertion tube of an endoscope itself.

Furthermore, it will be understood that the present invention is compatible with all types of instruments, including catheters, obturators, etc. Also, visualization devices used with the present access device are not to be limited to endoscopes, but also include all types of such devices, including fluoroscopes, etc. Thus, the terms "instrument" and "endoscope" are intended to be only illustrative and representative of the wide variety of devices that can be utilized in accordance with the present invention, and such terms are not intended to be limiting in any respect.

Thus, the fact that the present invention is described with respect to an introducer is illustrative only and not intended to be limiting in any respect.

### Surgical Introducer

Thus, with further reference to FIGURE 1, there is illustrated a surgical introducer 100 into which the principles of the present invention have been incorporated. In this case, the introducer 100 is intended for gynecological procedures, such as hysteroscopy or cystoscopy; however, again, a wide variety of procedures may be performed with the surgical access device 100 of the present invention.

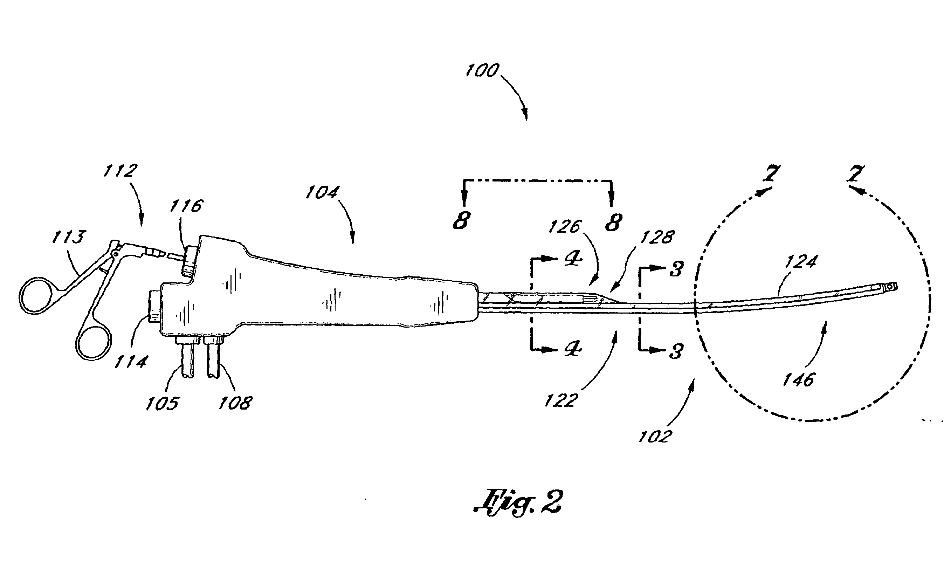

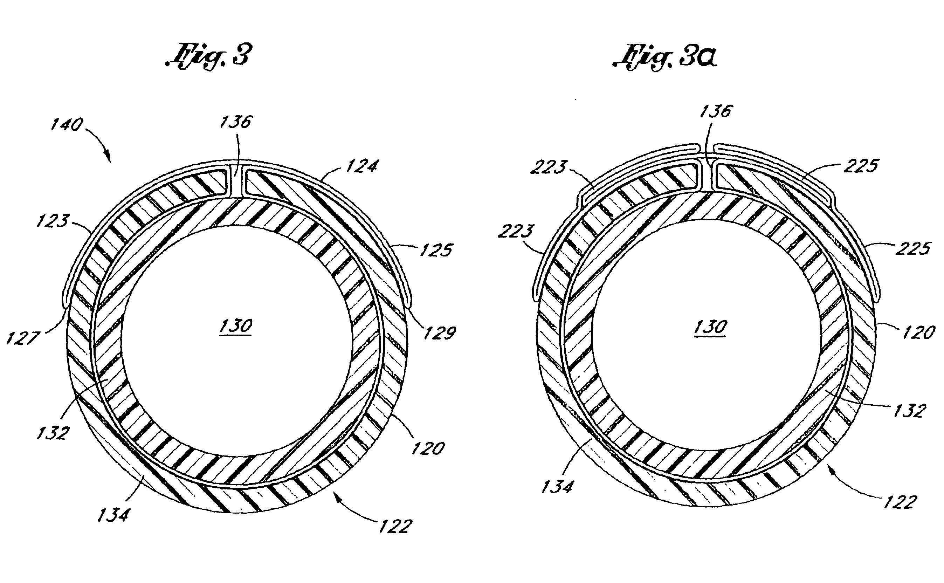

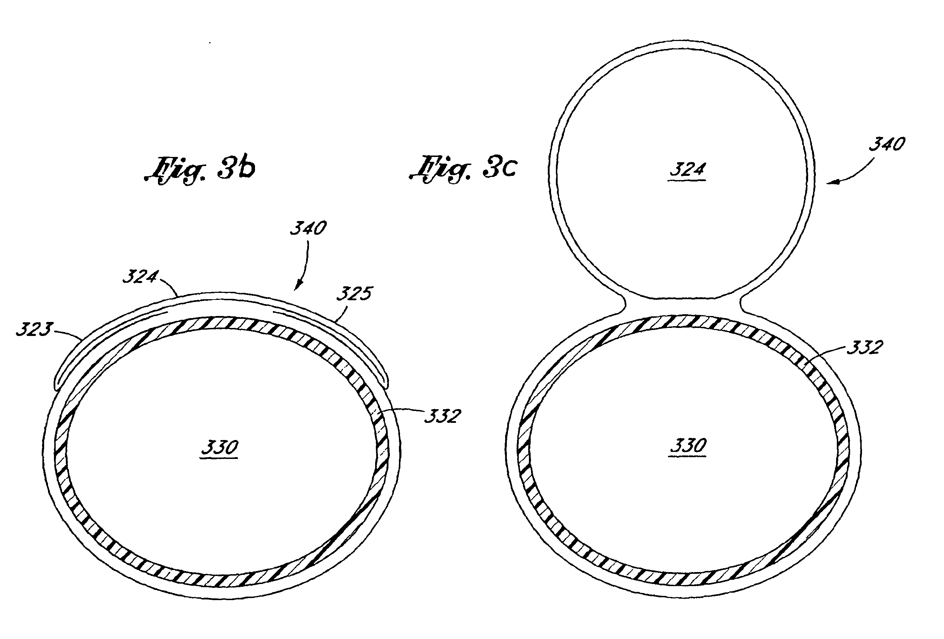

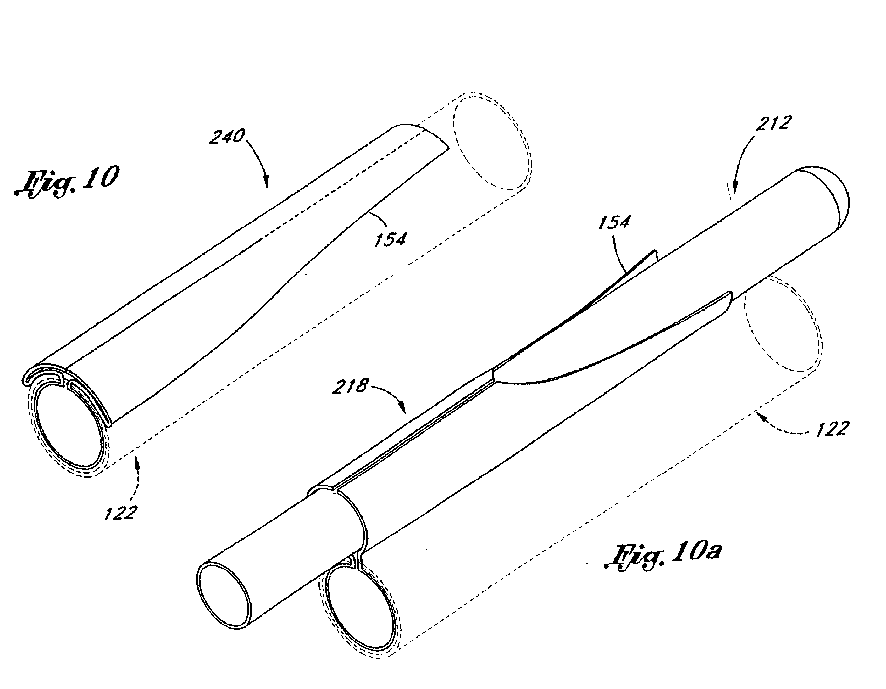

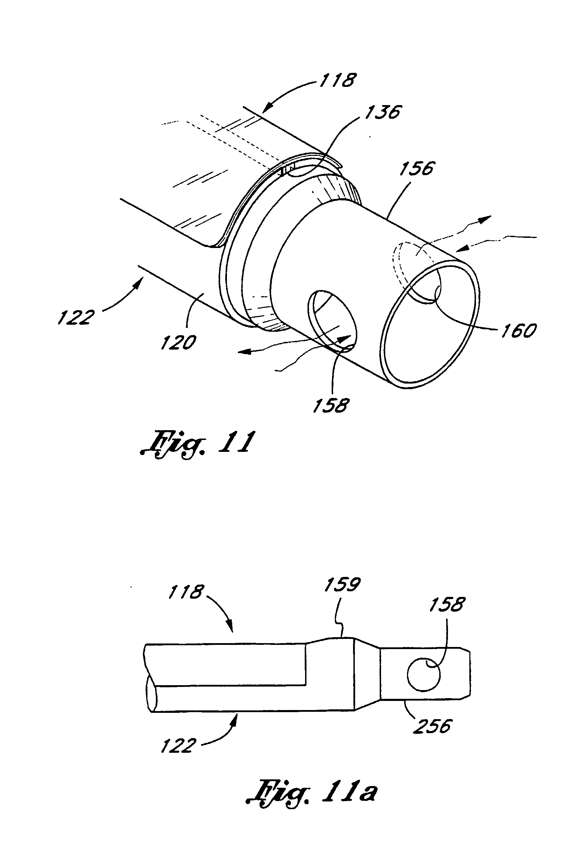

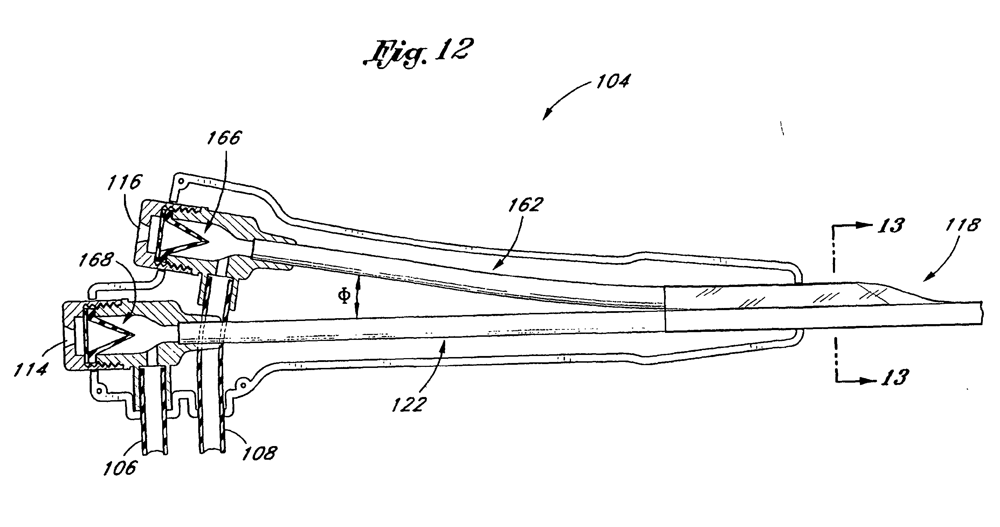

As shown in FIGURE 1, the access device 100 comprises a distal insertion portion 102, which is intended for insertion into the patient's body, and a proximal housing portion 104, which generally remains outside of the patient's body. In this case, access to the patient's body is achieved through dilation of the cervix; however, in other procedures, access may be gained through other natural openings in the body or by surgical incision, etc. The details of construction of the insertion portion 102 are described below in more detail and illustrated in connection with FIGURES 3 and 4, while the details of the housing portion 104, including in-flow and out-flow conduits 106, 108, are described and illustrated below in connection with FIGURE 12.

To the left of the proximal housing portion 104 as shown in FIGURE 1, there is shown in exploded relationship to the introducer 100 an endoscope 110 and a secondary instrument 112, in this case a grasper which can be used for the removal of foreign bodies or tissue. The endoscope 110 is shown partially inserted into a main or endoscopic port 114 formed at the proximal end of the introducer 100. The secondary instrument 112 is shown positioned prior to insertion into a secondary or instrument port 116. The terminology of main or endoscopic port 114 and secondary or instrument port 116 is merely illustrative since the endoscope 110 is typically inserted into the main port 114 of the introducer 100, while the secondary instrument 112 is inserted through the secondary port 116. However, in accordance with the principles of the present invention, this arrangement can be reversed, or any other of a wide variety of instruments may be used in connection with the various ports of the access device. In addition, multiple ports in addition to two may be formed on the introducer 100, depending upon the nature of the procedure to be performed.

FIGURE 1 illustrates the introducer 100 prior to and upon insertion into the patient's body, but prior to insertion of any secondary instrument. Although not readily apparent from FIGURE 1, a guide channel 118 of the present invention is mounted and formed on an exterior surface 120 of the endoscopic channel 122 which forms the basic cross-sectional profile of the insertion portion 102 of the introducer 100. Since the secondary instrument 112 has not yet been inserted through the instrument port 116 or through the proximal housing 104 to the insertion portion 102, the guide channel 118 for the instrument is virtually unnoticeable to the eye or touch. Thus, as illustrated in FIGURE 1, the guide channel 118 adds only a negligible dimension to the profile of the access device 100, thus minimizing pain and discomfort to the patient. This a particular advantage in gynecological procedures which are frequently performed on an out-patient basis and under only a local anesthetic. Thus, if a secondary procedure proves unnecessary, there has been no unnecessary discomfort or pain to the patient since the profile of the introducer 100 has been minimized. However, if such a secondary procedure becomes necessary, it can be readily accomplished with only a minimal intrusion into the body through the existing port, without the need to schedule a second surgery. It also has the obvious advantage of not requiring a pre-dilatation step prior to the insertion of the introducer 100. In some cases, such as for the cervix, dilatation of the cervical canal can be painful to the patient and should be minimized as much as possible. In addition, the uterine cavity requires distension to allow for proper visualization. If the cervix is over dilated during the pre-dilatation step, excessive cervical leakage can occur when trying to distend the uterus. Thus, the introducer 100 with its guide channel 118 and low profile will minimize pre-dilatation of the cervix and will provide a guide channel 118 with the diameter of the instrument being used, thereby reducing the leakage potential via an overly dilated cervical canal.

It will be noted from FIGURE 1 that the guide channel 118 closely conforms to the outer configuration of the endoscopic channel 122 without the need for outer sheaths or bands which would increase the profile thereof. Moreover, since the guide channel 118 is formed on the exterior 120 of the endoscopic channel 122, its natural lubricity provides an important advantage in connection with the ease of insertion of the introducer 100. However, it will be noted in accordance with the present invention that the guide channel 118 may also be formed on or within the endoscopic channel 122 in other configurations with respect to the introducer 100. Moreover, multiple guide channels may be formed on or incorporated into the main or endoscopic channel 122.

FIGURE 2 is a view of the introducer 100 of the present invention illustrating the deployment of the secondary instrument 112 as it advances along the guide channel 118. In this FIGURE 2, the guide channel portion 124 ahead of the instrument 112 is folded over and upon the insertion portion 102 of the introducer 100. The characteristics of the guide channel 118 at this location are described below in more detail in connection with the description of FIGURE 3. However, in the region of a leading edge 126 of the instrument, a guide channel portion 128 is shown to be releasing its insertion position and gradually expanding so as to allow the instrument 112 to advance. It will be noted that, although FIGURE 2 shows expansion or dilation to be achievable by the use of the secondary instrument itself, other instruments, hollow tubings, media, or means are equally available to achieve expansion in accordance with the guide channel 118 of the present invention.

As the instrument 112 advances, the guide channel 118 gradually releases in order to maintain the minimum profile of the introducer 100. Thus, pain and discomfort to the patient are minimized. Furthermore, the surgical access device 100 of the present invention makes it possible for more endoscopic surgical procedures to be conducted on an out-patient basis with only minimal or local anesthesia.

Such procedures would include those which are planned and scheduled, as well as secondary procedures which are unanticipated. In other words, a surgeon may enter a patient's body endoscopically for the purpose of a particular, planned diagnostic or therapeutic purpose. Initial insertion of the introducer 100 is typically accomplished under the visual guidance of the endoscope 110. However, once inside the patient's body, under these visual conditions, it may become necessary or desirable to perform a secondary procedure using a secondary instrument 112 inserted through the instrument port 116 and advanced through the proximal housing portion 104 and into the guide channel 118 of the present invention. Thus, regardless of the initial purpose of the endoscopic procedure, this secondary procedure can be performed virtually simultaneously without rescheduling a second procedure. Moreover, the secondary procedure can be performed with only minimal discomfort to the patient and without withdrawing or reinserting the endoscope or any other instruments. Because of the narrow profile of the present access device 100, a wide variety of primary and secondary endoscopic procedures (as well as multiple procedures of all types) can be safely and efficiently performed on an out-patient basis. Thus, the high cost of health care can be contained somewhat.

### Guide Channel

An important feature of the present invention which allows these advantages to accrue is the guide channel 118 of the introducer 100. This guide channel 118 can be described in more detail in connection with the cross-sectional drawings of FIGURES 3 and 4. FIGURE 3 is a cross-sectional view of the insertion portion 102 of the introducer 100 at a location ahead of the advancing instrument 112. FIGURE 3 illustrates the main or endoscopic lumen 130 for the insertion of the endoscope 110 or other instrument (although, the endoscope 110 is not shown in FIGURE 3 for clarity of illustration). This lumen 130 is formed by the endoscopic channel 122 which may comprise a tube 132 of various constructions. The endoscopic tube 132 is in turn surrounded by a larger diameter split tube or sheath 134. The split in the sheath 134 defines a slit or longitudinal opening 136. Sandwiched between the inner endoscopic tube 132 and the outer split sheath 134 is a membrane 140 which forms the present guide channel 118. This membrane 140 can initially be formed in the shape of a tube or other construction.

As shown in FIGURE 3, the membrane 140 surrounds the inner endoscopic tube 132 but, due to its greater diameter, also extends out of the longitudinal opening 136 in the split sheath 134. This excess membrane material may be folded back onto the outer surface 120 of the split sheath 134 to form a double-layer of the membrane 140 along a partial circumference of the introducer 100.

This folded-back portion 124 shown in FIGURE 3, forming pleats 123, 125, is that portion which defines the guide channel 118 for the instrument 112, as illustrated in more detail in FIGURES 2 and 4. However, as illustrated in FIGURE 3, prior to instrument deployment, the guide channel 118 is defined by a membrane which closely conforms to the outer surface 120 of the split sheath 134. For example, the lateral edges of the pleats 123, 125 can be provided with thin creases or seams 127, 129, which can be formed and set in the membrane material. Thus, the narrow profile of the introducer 100 is maintained. In addition, because of the close conformity of the guide channel membrane 140, it is less likely to be distorted or disturbed upon insertion of the introducer 100 into the body. Thus, the guide channel 118 maintains its structural integrity and avoids patient discomfort even before insertion of the secondary instrument 112.

The membrane 140 which comprises the guide channel 118 can be extremely thin, ranging in thickness between .0005" (.127 mm) and .002" (.508 mm), preferably being about .001" (.254 mm). Thus, even when doubled back on itself and lying on the outer surface 120 of the split sheath 134, the guide channel 118 adds only a negligible thickness to the profile of the surgical access device 100. Moreover, the guide channel in its pre-release position 124 shown in FIGURE 3 will hold a set in this position and does not require any external elastic sheets or strapping to bind it in position on the introducer 100.

It will be understood, as noted above, that the present guide channel 118 can be formed on or in connection with surgical access devices of a wide variety. Moreover, in its pre-release position 124 (which it assumes prior to and even during insertion of the access device 100 into the body, but prior to deployment of an instrument 112 through the guide channel 118), the guide channel 118 can be stored, wrapped, or folded in a number of configurations, other than that shown in FIGURE 3.

For example, FIGURE 3a shows multiple pleats or folds 223, 225 of pleated portion 224 in the membrane 140 which will facilitate a larger membrane channel 118 and can follow a multitude of folding patterns which preferentially unfold upon the exerted force of the insertion element. The additional material which constitutes these multiple folds 224 will allow for larger instruments to pass through the membrane channel 118. Likewise, the folding pattern of the pleats may be such that all of the pleat is on one lateral side of the introducer or the other, rather than the two equal pleats shown in FIGURE 3a.

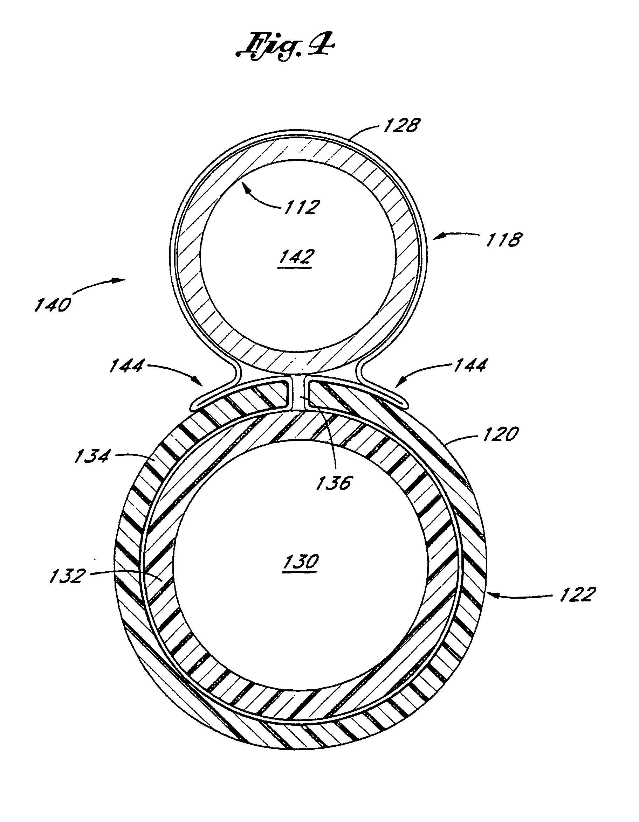

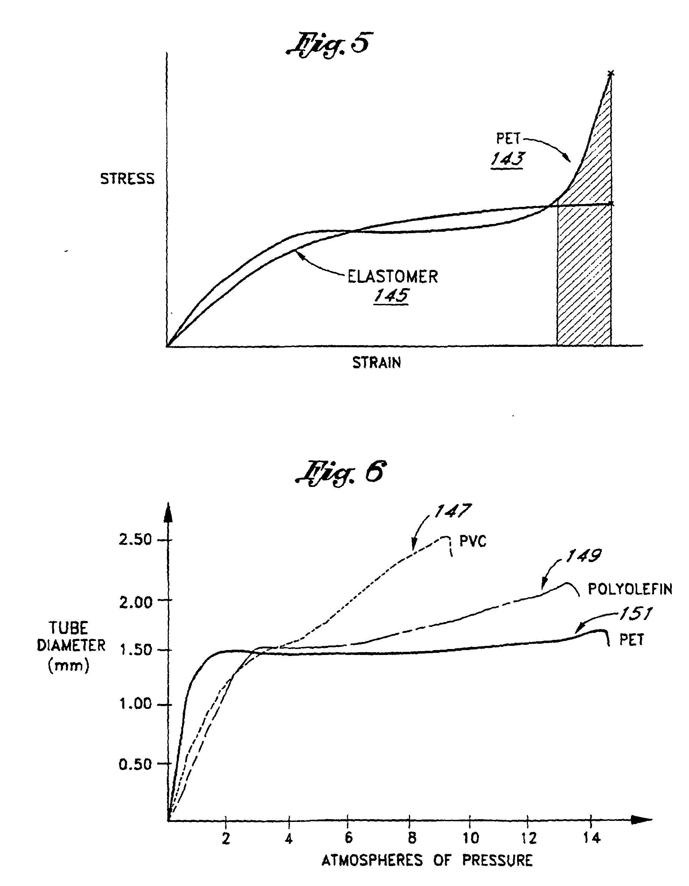

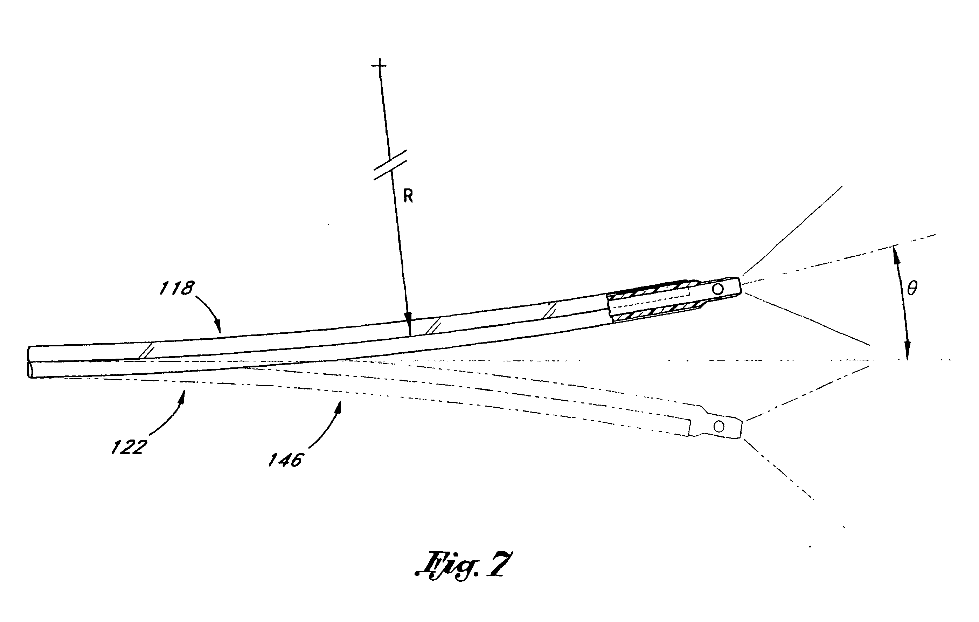

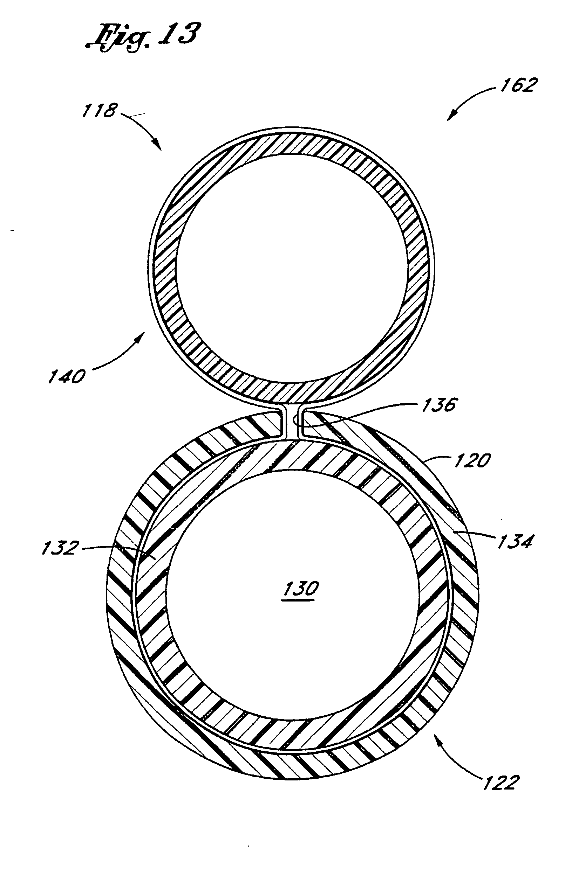

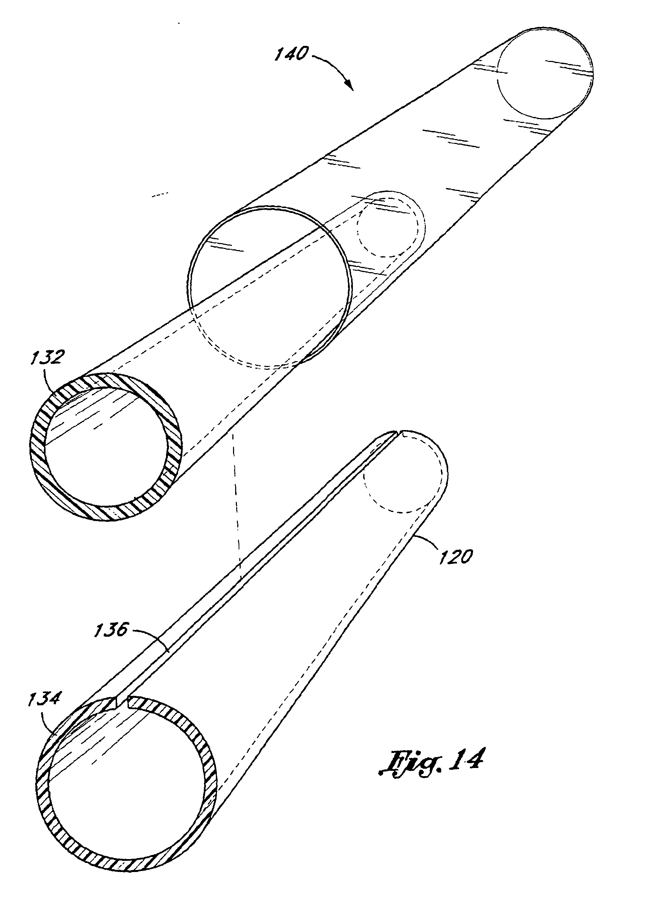

Although the method of construction of the introducer 100 of FIGURE 2 is shown and described in more detail in connection with FIGURE 14, it will be understood that the endoscopic tube 132 can be constructed from a wide variety of materials which provides rigidity and protection for the endoscope 110. Preferably, such a tube 132 can take the form of a stainless steel hypotube. Thus, the tube 132 can provide the rigidity necessary for initial insertion (especially in difficult procedures such as a laparoscopy), and can be used to move tissue without fear of damage to the endoscope 110 within. Moreover, the endoscopic tube 132 can take on a bend or curve, as illustrated in FIGURE 7, to facilitate a particular procedure. With advancements in rigid and semi-rigid endoscopes, such curves or bends in introducers can facilitate intricate navigational procedures while not damaging the endoscope. The curves and bends also direct the visualization area of the endoscope to preferentially view anatomical structures not on the axis of the insertion point in the body.

The outer split sheath 134, in its typical construction, is smooth and lubricous in order to facilitate insertion of the introducer 100. It may be constructed from a durable, bio-compatible polymeric material, such as nylon. Preferably, nylon 11 can be utilized.

It will be noted in connection with FIGURE 3 that typical introducer construction will include both the endoscopic tube 132 and the outer nylon layer 134. Thus, the guide channel 118 of the present invention does not significantly increase the profile of such an access device 100. In this connection, a number of cross-sectional introducer configurations will be readily apparent to those of ordinary skill in the art, including noncircular configurations. In addition, a wide variety of endoscopic tube and split sheath wall thicknesses are within the realm of the person of ordinary skill; however, preferably, the endoscopic tube 132 would have a wall thickness of approximately .008" (2.032 mm), while the split sheath 134 would have a wall thickness of approximately .005" (1.27 mm).

On the other hand, the versatility of the guide channel of the present invention allows it to be incorporated into introducers of even narrower profiles. For example, FIGURE 3b illustrates a cross-sectional view of an alternate embodiment of the present introducer which does not utilize an outer split sheath to capture a guide channel membrane 340 onto a stainless steel hypotube 332. In this case the guide channel membrane 340 can be heat bonded or otherwise coupled to the tube 332 by adhesive or other means. To facilitate this construction, the guide channel membrane 340 can be supplied in a multi-lumen or figure-8 configuration, as illustrated in FIGURE 3c, with one lumen 330 of the membrane 340 being mounted on the hypotube 332, leaving the second lumen 342 to be folded or pleated thereabout to provide a guide channel 324 in the stored position having pleats 323, 325. Thus, FIGURE 3c illustrates this mounting position prior to the folding of the membrane 340, which position is shown in FIGURE 3b. It will also be noted in connection with the embodiment of FIGURES 3b and 3c that guide channel membranes having multiple lumens can be provided and mounted on the hypotube in this or another manner, and then folded and set in position about the tube in order to provide an introducer with extremely narrow profile. Thus, three, four, or more membrane tubes can be mounted, either jointly or separately, on the introducer so as to provide multiple lumens. In addition, one or more of the tubes (which can be constructed from an extrusion or other process) may be collapsible, while others may be noncollapsible, due either to the wall thickness of the membrane extrusion or some other rigidifying or reinforcing mechanism (such as a hypotube or the like).

With reference to FIGURE 4, there is shown cross-sectional view of the present introducer 100 through the insertion portion 102 where the instrument 112 has already advanced. In this case, the instrument 112 almost completely occupies a lumen 142 defined by the guide channel 118 of the present invention. As shown in FIGURE 4, the guide channel 118 is shown releasing its predeployment position to allow the instrument 112 to easily pass along the guide channel 118 and into the patient.

The guide channel 118 of the present invention may be constructed from a membrane 140 which exhibits a number of advantageous characteristics. For example, in addition to its external lubricity, the membrane 140 is also internally lubricous to facilitate the deployment of the instrument 112. The physical characteristics of the membrane 140 also allow the guide channel 118 to be self-adjusting. That is, as shown in FIGURE 4, the guide channel 118 releases only to the extent necessary to accommodate the particular instrument being inserted through it. If not needed, the membrane 140 remains folded in its set position in the region indicated by arrows 144 of FIGURE 4. This advantageous characteristics allows the guide channel 118 to accommodate a number of instruments of various cross-sectional dimensions without significantly increasing the profile of the introducer 100.

Likewise, if the instrument 112 is removed from the guide channel 118, the membrane 140 causes it to maintain approximately the same position as it did with the instrument 112 inserted within, thus facilitating reinsertion of the same instrument or another instrument. For instance, in this case of removing multiple portions of tissue from the body, it may be necessary to remove tissue and then reinsert the instrument 112 back through the guide channel 118 to remove more tissue. Thus, there is no radial resistance to reinsertion which reduces the risk of damage to the guide channel 118 and discomfort to the patient. If the instrument 112 is withdrawn upon completion of the procedure, the guide channel 118 readily conforms to the external forces applied to it by the surrounding tissue, thereby causing the guide channel to collapse upon withdrawal of the introducer 100, so that pain or trauma to the patient is avoided. Also, if desired, the guide channel 118 can be evacuated of fluids in order to facilitate its collapse prior to withdrawal.

As noted above, the guide channel membrane 140 is noncompliant both longitudinally and radially. Thus, it does not exhibit elastic characteristics which might cause the guide channel 118 to bunch up or bind as the instrument 112 is advanced therethrough. Moreover, the guide channel membrane 140 is malleable, meaning that it tends to conform to the pressures and forces exerted upon it by ambient conditions, including anatomy, tissue, and other media. This feature advantageously tends to reduce resistance to movement of the access device 100 and enhance patient comfort. On the other hand, in the absence of such ambient forces, the membrane 140 maintains its position and configuration, having somewhat of a "memory" in this regard and exhibiting patentency characteristics. In other words, the membrane can be self-supporting, defining an instrument lumen without the need for internal wall support means.

A number of materials can achieve these advantages of the guide channel membrane 140 of the present invention. For example, inelastic polymers or other pleated, woven, or braided materials can be utilized. Preferably, however, highly orientated or cross-linked, noncompliant polymers can be utilized as a guide channel membrane material. Such materials tend to be thermoplastically settable, with glass transition temperatures greater than room temperature. In addition, such polymers are semicrystalline and deformable in the crystalline state.

These properties yield certain advantages in connection with the present invention. For example, in order to minimize profile, the guide channel membrane can be very thin, yet strong. Because the material is deformable in its crystalline state, it may be folded, pleated, rolled, or otherwise stored in combination with the introducer without the loss of its advantageous mechanical properties, such as strength, noncompliance, etc. In achieving this storage position, the membrane can readily conform to the surface or space in which it is placed. Because the membrane is thermoplastically settable, once it is stored in this position, the application of thermal energy will result in the realignment of the crystalline structure so that the membrane retains its storage position; however, it will be understood that this set in the membrane storage position can also be achieved by the application of mechanical energy or chemicals (e.g., adhesives).

Thus, it can be said that the membrane is "self-conformable," since it can conform to a surface or space and assume a given configuration, and then be self-retained in that configuration. In addition, once it is desired to remove the membrane from its stored configuration (e.g., in order to achieve dilation), its deformability is again an advantage. Besides thermal energy, mechanical, electrical, chemical, or pneumatic energy can be applied to deploy the guide channel. Again, because it is deformable in its crystalline state, it will tend to become set or retained in its deployed configuration.

One preferred polymeric example of the present guide channel membrane is polyethylene terephthalate ("PET"), although other polymers are possible. For example, polybutyl terephthalate may be utilized as a guide channel 118, as well as nylon 6 or nylon 66. These materials, as well as others, exhibit the advantages described above.

In the case of PET, FIGURE 5 illustrates the noncompliant (stress/strain relationship) of oriented PET as compared with a typical elastomer. A PET curve 143 in the graph shows a shaded region which depicts the behavior of a highly-orientated PET which has been pre-stretched. These stress-strain properties relate to a material which has high strength and very little elongation when a load is exerted upon it. Conversely, an elastomer behaves different in this and all sections of its curve 145 on the graph by elongating with little additional stress. Thus, where careful precision in the configuration of the guide channel is necessary, membrane material such as PET is advantageous.

In addition, FIGURE 6 illustrates the relationships between tube diameters and internal pressure for tubes comprising PVC 147, polyolefin 149 and PET material 151. In the graphs in FIGURE 6, plots are shown for a closed vessel structure such as a balloon for various materials in which the outer diameter measurement is plotted versus the internal pressure applied. In this example, the PET material 151 demonstrates very little distension with greater internal pressure as a result of its high strength and low elongation in comparison to the polyolefin and PVC materials 149, 147 used for this example. Nevertheless, these and other materials can provide suitable guide channel membranes if treated properly during their manufacturing process. Thus, a major component of the inelastic behavior of PET and polyolefins is the fact that their polymer chains in the material have been highly orientated (PET) or cross-linked (polyolefins) with each other, providing greater strength and resistance to strain. This high degree of orientation, either by processing or secondary operations such as radiation treatment or forming and axial stretching, enables the nondistensible behavior of the present guide channel membrane.

### Curved Guide Channels

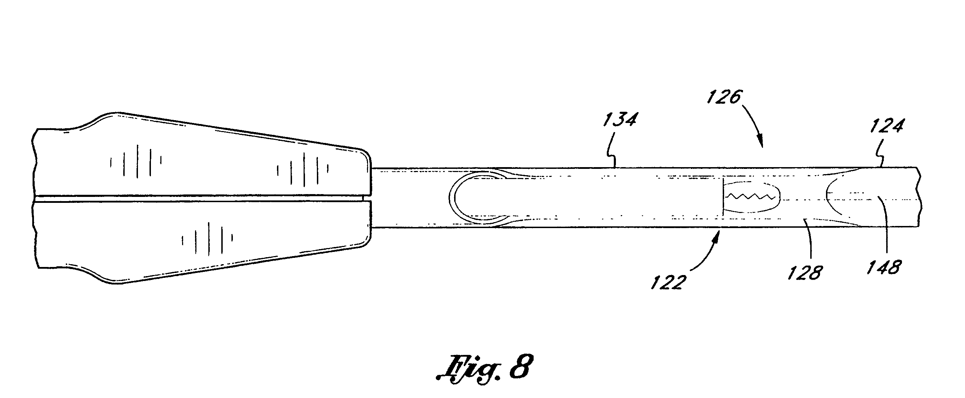

FIGURES 7 and 8 illustrate another advantage of the present guide channel 118 relating to its ability to actually guide a rigid or semi-rigid instrument being deployed through it along a curved path. This advantage can best be described in light of the following background information. As shown in FIGURE 1, a distal end 146 of the introducer 100 of the present invention is curved slightly. This curvature provides certain advantages, depending upon the endoscopic surgical procedure being performed. In this case, the introducer 100 of FIGURE 1 can be used in performing a hysteroscopy, wherein an angle of curvature θ in the range of 5-15° in the introducer 100 is advantageous in navigating the uterine canal. This curvature, as noted above, can also be used to gently move tissue out of the way in order to achieve advancement of the endoscope/introducer combination 110/100 to the desired location. With recent advancements in endoscopes, curvatures in these ranges, and even up to 30°, can be achieved without damage to the optical systems of the endoscopes.

However, another advantage of a curved introducer is illustrated in FIGURE 7. As shown therein, an endoscope 110 which provides vision out of the curved distal end 146 of an introducer is able to sweep out a larger field of vision upon rotation of said introducer 100. This is illustrated in FIGURE 7 by a first and second position (in phantom) of the distal end 146, rotated 180 degrees apart. This is a particular advantage in the case of rigid or semi-rigid endoscopes which do not have articulation means mounted at the distal end. In order to achieve this degree of curvature, as noted above in connection with FIGURES 3 and 4, the endoscopic tube 132 can be constructed from a strong and rigid stainless steel material which can be preformed or bent to the desired curvature. While this material wiii provide the rigidity, strength, and protection for the endoscope 110, it does not in and of itself solve the problems of secondary channels formed in such curved surgical access devices. Thus, secondary channels of the prior art were constructed to be used only with straight instruments or flexible instruments, such as catheters.

The guide channel 118 of the present invention accommodates a rigid or semi-rigid instrument which experiences a curve along its shaft as it is inserted through the guide channel. Thus, the guide channel 118 of the present invention makes possible another category of endoscopic surgical procedures requiring a curved but rigid or semi-rigid instrument. First, as noted above, the guide channel membrane 140 is constructed from an extremely strong material in order to withstand the stresses on it caused by a biased instrument mounted therein. Nonetheless, it is important that the secondary instrument being advanced through the guide channel 118 is provided with a smooth and straight passage. Any slippage or lateral movement may cause damage to the guide channel 118 and/or discomfort to the patient.

Moreover, the guide channel 118 may be located at a number of different circumferential locations with respect to the main or endoscopic channel 122. Thus, as shown in FIGURE 7 in phantom, the guide channel 118 is located at the bottom of the main channel curvature. This longitudinal location may be considered "inboard" with respect to the curvature of the introducer 100. However, the guide channel 118 may also be positioned, as needed or desirable for a particular procedure, "outboard" of the introducer curvature (e.g., on the upper portion of the introducer 100 as oriented in accordance with FIGURE 7) or "sideboard" (e.g., on one or more lateral sides of the introducer 100). At any of these various locations, the curved instrument will exert, due to the bias or spring force it causes as it bends, to apply a force on the outer sheath 134 of the main channel 122 and/or the guide channel 118.

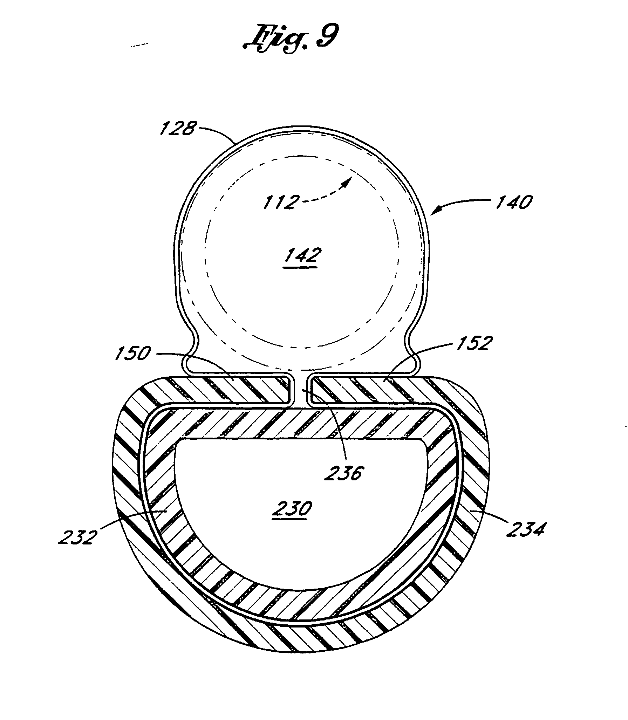

Thus, as illustrated in FIGURE 8, the guide channel 118 of the present invention is provided with a guide platform or other type of guide rail 148 in order to actually guide the instrument 112 in its path along the guide channel 118. Thus, even though the instrument may be bending and flexing, it will tend to be retained in its path along the guide platform 148, which acts as a rail or track for the instrument to follow. As illustrated in FIGURE 8, in one preferred embodiment the guide rail 148 comprises the slit 136 in the split sheath 134 of the main endoscopic channel 122. The longitudinal guiding capabilities of this slit 136 are also illustrated in FIGURE 4. However, other guide platform configurations are possible, as illustrated in FIGURE 9. FIGURE 9 depicts a D-shaped main tube 232 which has a flattened area 150, 152 for instrument travel. This flattened area could also contain recesses or slots (not shown) to further facilitate directed insertion of the instrument. In any particular guide channel 118 and guide rail 148 configuration, the guide channel membrane 140 is able to assume a number of storage or set positions, as described above.

Thus, as illustrated in FIGURE 9, the guide platform 148 may be flattened or may be provided with lateral walls 150, 152 in order to provide sure guidance for the curved instrument. These lateral walls may also have upward projections (not shown) so as to contain more securely the sides of the instrument 112. Therefore, in accordance with an important advantage of the present invention, the surgical access device can be used to guide an instrument along a specified path with respect to the main channel 122 so that it achieves accurate placement with respect to a specific location at the distal end of the access device 100.

### Distal Portion

FIGURES 10 and 10a illustrate an alternate embodiment of a guide channel 218 of the present invention, which is characterized by a perforated or serrated guide channel membrane 240. As noted in FIGURES 10 and 10a, the guide channel membrane 240 is provided with a reduced diameter in the region of its distal tip 154. However, in this region or at other regions along the longitudinal length of the guide channel 218, the membrane 240 is perforated or serrated in order to facilitate its release as a secondary instrument 212 is advanced. Furthermore, the perforations or slits can be such that the guide channel 218 opens up completely in order to allow instrument access to the lateral regions of the access device. Such perforated or serrated guide channels 218 also provide other guide channel storage options, as well as other advantages which will be apparent to one of ordinary skill.

FIGURE 11 illustrates one embodiment of a distal tip 156 of the present introducer 100. In this embodiment, a tapered distal profile of the access device 100 facilitates the insertion process; however, the extreme distal edge is rounded or blunt in order to avoid damage to the tissue. Holes 158, 160 shown in the distal tip 156 are necessary to provide aspiration and avoid choking of irrigation media. In this embodiment, the guide channel at the distal tip 156, which is shown folded back on the outer surface 120 of the split sheath 134, can be securely sealed to the body of the introducer 100 by a heat seal process. Thus, the guide channel 118 is sealed and is impervious against contamination or distortion as the access device 100 is introduced into the body. An alternative embodiment of the distal tip 256 is shown in FIGURE 11a. The distal end of the introducer can be shaped so as to have an annular mound 159 at the distal tip of the guide channel 118 and just proximal of the distal tip of the access device 100. Likewise, this mound 159 can serve to protect the distal end of the guide channel 118 against contamination or damage as the access device 100 is introduced into the body and navigated through its various anatomy.

### Proximal Housing Portion

As explained above in connection with FIGURE 1, the proximal housing portion 104 of the present invention surrounds the main endoscopic channel 122 and a merge channel 162 positioned above it in FIGURE 12. The merge channel 162 allows an endoscope 110 or secondary instrument 112 to be inserted through it for deployment through the guide channel 118, shown in its predeployment position in FIGURE 12. The merge channel 162 also allows a proximal handle 113 of the instrument 112 to be offset or displaced with respect to any endoscope or instrument inserted in the main channel 122 in order to facilitate use of the access device 100 by the surgeon.

With reference to FIGURES 12 and 13, the proximal housing portion 104 of the surgical access device 100 of the present invention can be described. FIGURE 12 is a partial cross-sectional view of the side of the access device 104, while FIGURE 13 is a cross-sectional view taken along lines 13-13 of FIGURE 13 in which is illustrated the piggyback arrangement of the main or endoscopic channel 122 and the merge channel 162 of the housing 104.

With reference to FIGURE 12, it will be seen that the merge channel 162 converges upon the main channel 122 at a shallow angle φ, gradually becoming asymptotic or tangential thereto. The merge angle φ should be sufficient to allow a slight bending or curvature in the instrument being inserted through the merge channel 162 and into the guide channel 118; preferably, an angle φ of about 4-30° is satisfactory with 11° preferable.

At the extreme proximal end of the housing portion 104, there is shown an inflow conduit 106 associated with the main channel 122 and an outflow conduit 108 associated with the merge channel 162. Depending upon the procedure being performed, the inflow conduit 106 may be utilized to pass distention or irrigation media down the main channel 122 to the distal end of the access device 100. Having the distension media run through the main tube 132 and around the endoscope 110 also allows for fluid to travel across the optics at the distal end of the endoscope 110 keeping this area free of blood and debris thereby improving visualization. The outflow channel 108 can be used to provide aspiration or other evacuation of fluids. Also, of course, the function of these conduits 106, 108 can be reversed or utilized in connection with multiple channels, as the case necessitates. In each case, duckbill valves 166, 168 are formed at the main or endoscopic port 14 and at the instrument port 116 in order to prevent the escape of fluids prior to insertion of the instruments into these respective channels. Likewise, 0-ring structures or washer elements prevent the escape of fluids around an endoscope or instrument when inserted through the ports 114, 116. However, it will be understood that other types of valving and conduit mechanisms can be utilized in connection with the present access device.

The cross-sectional view of FIGURE 13 also illustrates the association of the guide channel membrane 140 with respect to the merge channel 162. That is, the membrane 140 is shown extending through the slit or neck 136 in the split sheath 134 and completely around the merge channel 162. Advantageously, the guide channel membrane material can be heat formed onto both channels in order to provide some rigidity and strength to the proximal housing portion 104 of the access device 100. In fact, the membrane 140 can be extended proximally any desired distance, as shown in FIGURE 12.

### Method of Construction

FIGURE 14 is an exploded view of the present access device, illustrating a method of construction of the guide channel 118. Preferably, the main tubing 132 is preformed into the desired configuration, including any curvature. The guide channel membrane 140, which may take the form of an extruded tubing, as illustrated in FIGURE 14, or other configuration, is then placed eccentrically around the main tubing 132. The nylon split sheath 134 is then mechanically expanded so as to surround the main tubing/membrane material, thereby capturing the membrane 140 around the tubing 132. During this process the excess membrane material is allowed to protrude through the slit or neck 136 in the split sheath 134 and is then folded or stored with respect to the access device 100 in accordance with various configurations discussed above. Covers or forms are placed onto the folded membranes and closely conform to the profile of the introducer 100. These elements keep the membrane 140 in close configuration prior to heat setting. Another method of forming a closely fitting mold is to swell an elastomeric tubing, such as silicone, with Freon. Prior to swelling, the silicone tubing is of a smaller diameter than the introducer 100. Once swollen and enlarged, the silicone tubing is slid over the folded membrane and introducer. When the Freon evaporates, the silicone tubing will resume its pre-enlarged state thereby creating a tightly fitting mold over the membrane. After heat setting, the silicone tubing can be removed from the introducer leaving the membrane in a tight configuration with the introducer, in any case, a moderate amount of heat is applied to the access device in order to thermoplastically set the guide channel membrane in its stored position. In one preferred embodiment, the PET material which comprises the guide channel membrane has a glass transition temperature of 180°F (82.2°C). Thus, the setting temperature used in this method of construction is preferably about 160°F (71.1 °C). It will be noted in this regard that sterilization of the system is achieved at about 140°F (60°C).

The present method is not limited to that illustrated in FIGURE 14 or described above. A number of other methods of construction will become apparent to those of ordinary skill. For example, because of the thermoplastic nature of the membrane material, heat forming, heat staking, or heat shrinking can easily be employed in other aspects of the construction method. Although most costly, adhesives or other mechanical fasteners can be utilized. Some adhesive systems can be effectively incorporated into the design of the membrane material, main tube or slit sheath by making these tubes as a co-extrusion with a secondary bonding material as a composite within the tubing material or body in which thermal bonding techniques can be employed. Heat-activated or hot-melt adhesives, UV cured adhesives, or pressure-sensitive adhesive systems can also be used to facilitate attachment of the membrane channel, such as the embodiment of FIGURES 3b and 3c, wherein the membrane is mounted directly on the hypotube. Such techniques can also be used for keeping the folded membrane tacked down onto the surface of the main tube.

### Method of Use

In accordance with the operation of the present invention, a method of use comprises the steps of inserting a surgical access device into an opening in the patient's body (this step simultaneously comprising the insertion of an endoscope or other visualization device or other instrument into the body through said surgical access device or sequentially inserting the visualization device through the surgical access device and into the body after the surgical device is already in position), storing in connection with the body of the surgical access device one or more secondary guide channels and setting said guide channel in its storage position so as to provide a surgical access device including the guide channel with the profile which is substantially equivalent to the profile of the surgical access device without the guide channel, which may include visualizing by means of the endoscope the internal anatomy or tissue of the patient, and releasing the guide channel from its storage position with respect to the surgical access device so as to permit insertion into the patient's body of a secondary surgical instrument (or an endoscope or other visualization device), said releasing step being performed simultaneously with the insertion of said secondary instrument. The method may also comprise the steps of providing in connection with the guide channel a platform, rail, or track and guiding said secondary instrument through the guide channel along said platform, rail, or track. Clearly, because of the versatility of the surgical access device of the present invention, a number of methods of use are available.

### Open Loop Introducer

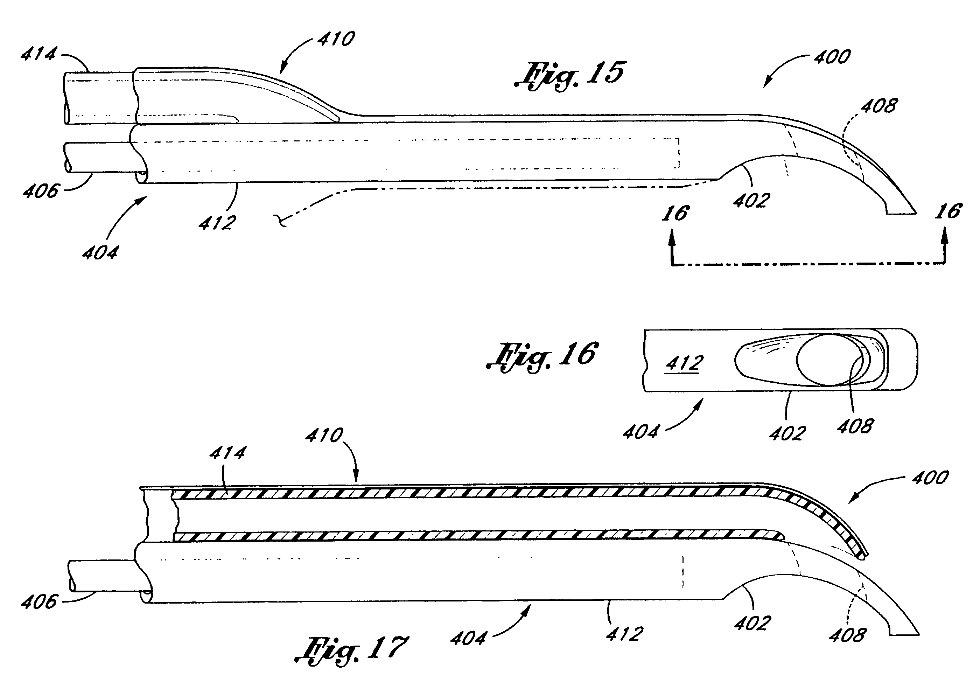

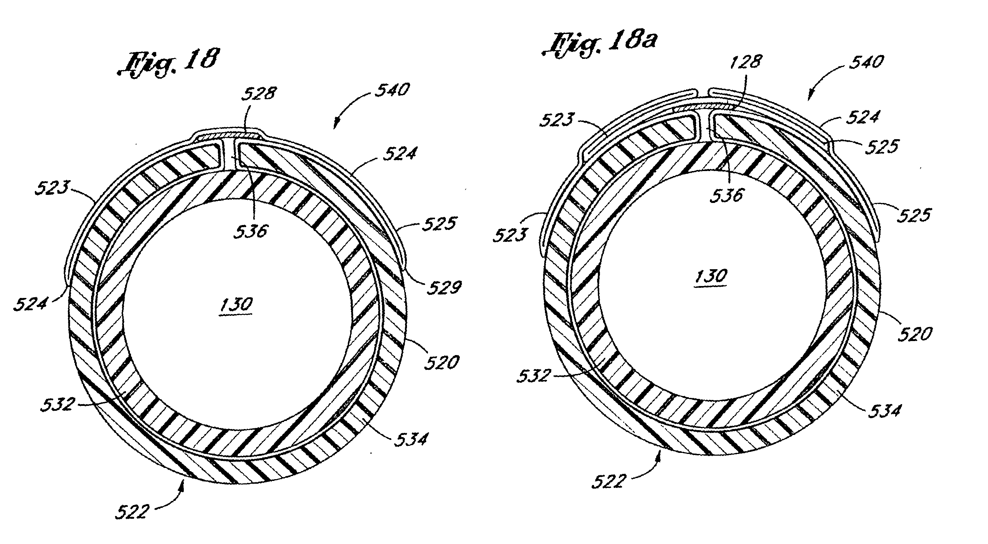

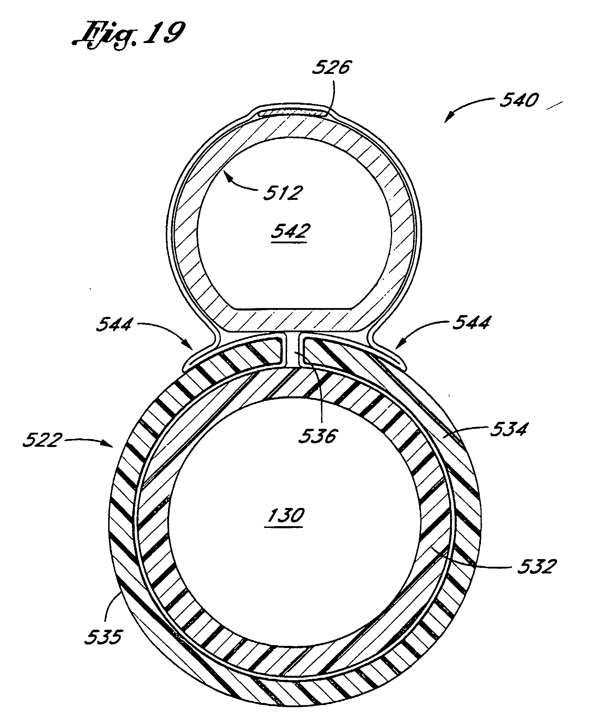

An alternate embodiment of a distal tip 400 of an introducer for biopsies is shown in FIGURES 15-17. As shown, an open-loop device 402 is formed on a curved distal tip of a main channel 404. Using an endoscope 406 positioned in the main channel 404, the device 402 may be manipulated by the physician to collect a tissue specimen. A hole 408 formed in the distal tip 400 provides a greater viewing field for the endoscope 406 of the tissue sample area. A guide channel 410 is preferably provided in an outboard configuration, and the device 402 has a curved length preferably extending past an inboard side 412 of the introducer. Alternatively, the guide channel 410 may be formed on the inboard side (see FIGURE 15 in phantom). Further, the length of the curved device 402 may be shorter so as not to extend past the inboard side 412 of the introducer. During endoscopic surgery, as discussed in greater detail below with regard to the method of use of the present device, an aspiration tube 414 may be provided in the guide channel 410 as shown in FIGURES 15 and 17 for removal of a tissue specimen comprising either separate cells or a tissue section.

It should also be noted, with regard to the introducer of FIGURES 15-17, that although the distal tip is shown without particular shaping for sealing the guide channel and such as described heretofore, it is understood that other shapes for sealing at the distal tip of the introducer may be readily incorporated.

In the open-loop device embodiment 402 being provided on the main channel of the introducer, the endoscope is inserted into the main channel and its field of vision is enhanced by the hole 408 in the distal tip of the main channel. An aspiration tube inserted through the guide channel removes blood at the viewing area or may be used to remove the specimen. An inboard or outboard configuration of the guide channel may be utilized. Alternatively, the endoscope and aspiration tube may be switched, so that the endoscope is in an inboard guide channel, for example.

### Laparoscopic Surgical Introducer

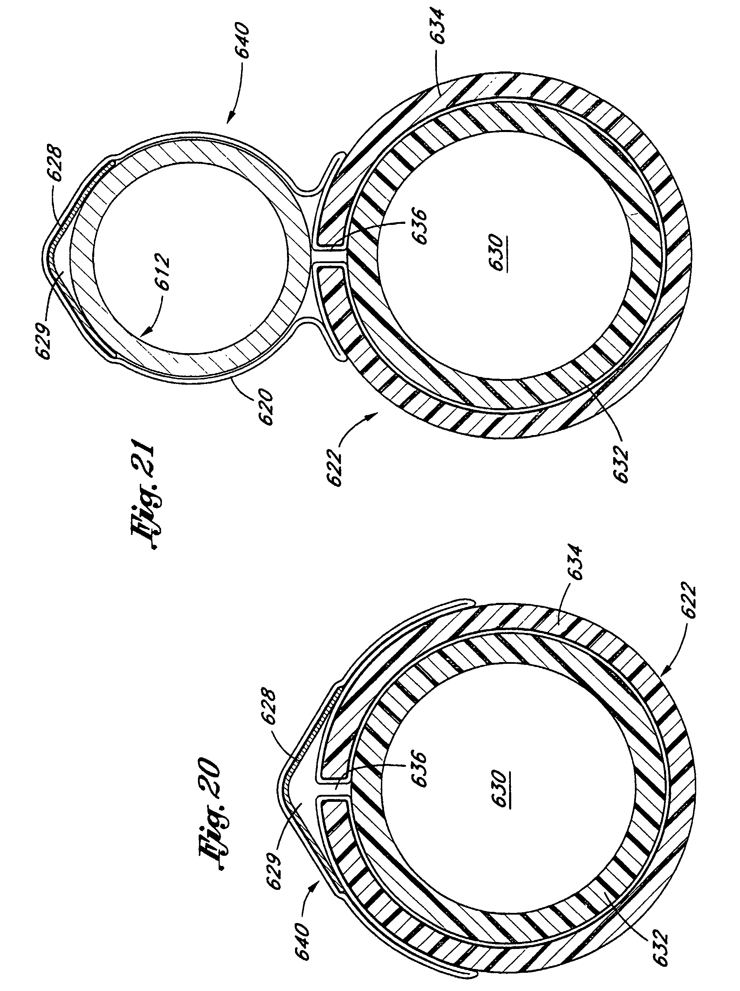

In another embodiment of the present access device, a laparoscopic surgical introducer is illustrated in FIGURES 18-22. In this embodiment, the introducer is intended primarily for laparoscopic procedures, such as cholecystectomy, bipolar tubal sterilization, gamete intra-fallopian transfer ("GIFT"), directed biopsy, etc. However, a wide variety of laparoscopic procedures may be performed with the laparoscopic introducer of the present invention.

FIGURES 18 and 18a illustrate the cross-sectional view of an alternative embodiment of the access device as taken along Line 3-3 in FIGURE 2. Due to the forces necessary to dilate the guide channel 540, particularly during a laparoscopic procedure, an alternate embodiment of the present introducer comprises a guide channel 540 with a reinforcing device 528 positioned therein. As shown in FIGUREs 18-19, this reinforcing device 528 can preferably take the form of a flat wire or other force distribution mechanism so that the dilation forces do not cause damage to the guide channel membrane. This reinforcement device 528 can also facilitate insertion of the dilator and other secondary instruments by providing a smooth riding surface. Moreover, the reinforcement device 528, in combination with other guide channel designs, can provide enhanced guiding and tracking characterisrics, as described in more detail below in connection with FIGUGRES 20-21.

Preferably, the present reinforcement device 528 takes the form of a flat wire having a thickness of between about .001-.004 inches (2.54 x 10⁻⁵m - 10.16 x 10⁻⁵m), and having a width of between about .040-.300 inches (10.16 x 10⁻⁴m - 7.62 x 10⁻³m). The tlat wire is preferably constructed from stainless steel or other sturdy material. The flat wire is positioned longitudinally along the laparoscopic channel 527, but within the folds of the guide channel 540. Thus, the dilator or secondary instrument will ride along the reinforcement device 528. To facilitate instrument insertion, the reinforcement device extends proximally along the merge channel so that the instrument rides below it as the instrument emerges from the distal end of the merge channel and begins to deploy the guide channel.

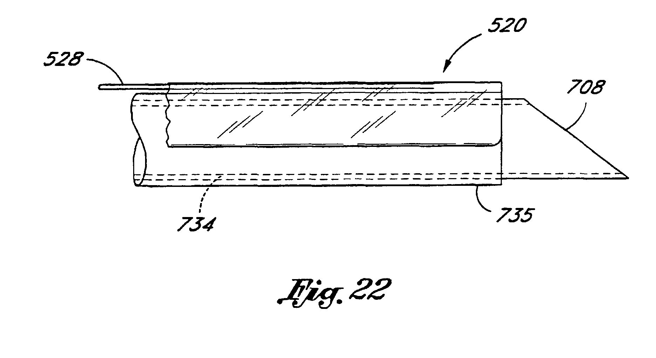

With reference to FIGURE 19, there is shown cross-sectional view of the laparoscopic embodiment of the present introducer through the insertion portion where the instrument 512 has already advanced. In this case, the secondary instrument 512 almost completely occupies a lumen 542 defined by the guide channel 540 of the present invention. As shown in FIGURE 19, the guide channel 540 is shown releasing its predeployment position to allow the secondary instrument 512 to easily pass along the guide channel 540 and into the patient.

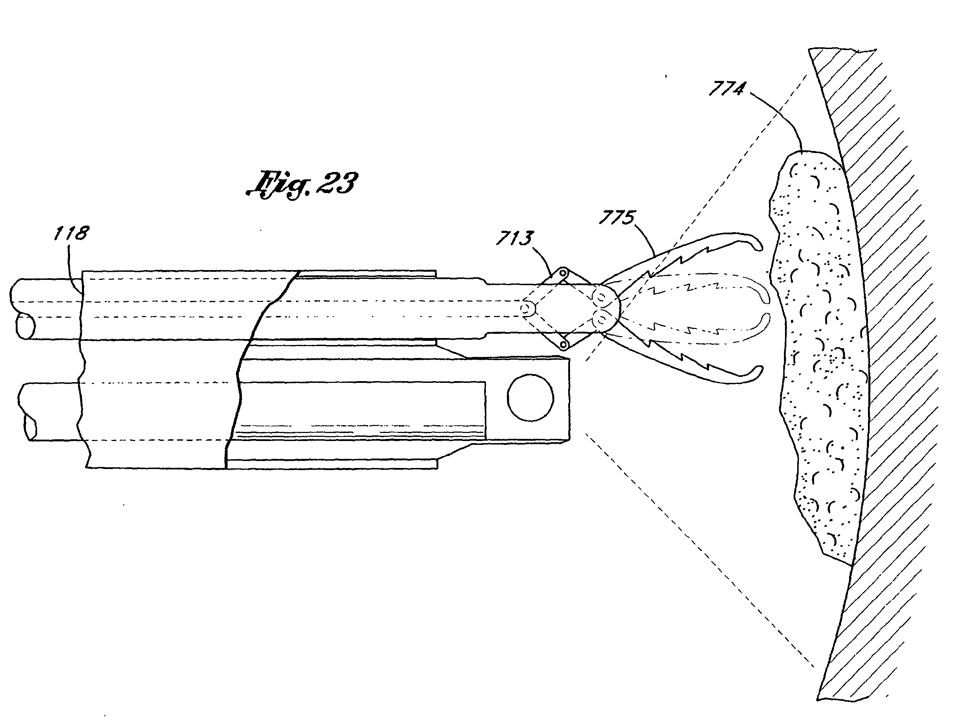

FIGURES 20-21 illustrate other features of the alternate embodiment of the guide channel 540 including its ability to securely guide the dilator 512 or instrument into a specific location with the patient's body. Referring to FIGURE 20, there is shown a cross-section of a nondilated guide channel 640 including a concave reinforcement device 628. In this case, the internal flat wire which forms the reinforcement device 628 is provided with a concave depression or recess 629 which produces additional guidance for the dilator and/or secondary instrument. This recess 629 serves to keep the instrument from "walking" off the laparoscopic channel 622 or otherwise deviating from its longitudinal path along the insertion portion of the introducer.

As shown in FIGURE 21, the recess 629 formed in the flat wire 628 can, in combination with the slit 636 in the split sheath 634, form a track for the secure guidance of the dilator or instrument. Thus, in this embodiment the instrument 612 is not likely to move laterally along its path, which movement would cause pain and discomfort to the patient and possible damage to the guide channel 640. Certainly, a wide variety of guide channel tracking designs are within the scope of the present invention. Moreover, guide rails (not shown) in addition to the slit 636, can be provided to provide even greater instrument guidance. These guide rails may be provided with upward projections (not shown) in order to provide even more secure guidance to the sides of the instrument.

The guide channel 640 of this embodiment of the present invention accommodates a rigid or semi-rigid instrument which may be inserted through the guide channel. As noted above, the guide channel membrane 620 is constructed from an extremely strong material in order to withstand the stresses on it caused by a instrument advancing therethrough. Nonetheless, it is important that the secondary instrument being advanced through the guide channel 640 be provided with a smooth and straight passage. Any slippage or lateral movement may cause damage to the guide channel 640 and/or discomfort to the patient.

Moreover, the guide channel 640 may be located at a number of different circumferential locations with respect to the main or endoscopic channel 622, and the main channel 622 may be curved. Thus, the guide channel 640 may be located at the bottom of the main channel curvature, which could be considered "inboard" with respect to the curvature of the introducer. However, the guide channel 640 may also be positioned, as needed or desirable for a particular procedure, "outboard" of the introducer (e.g., on the upper portion of the introducer), or "sideboard" (e.g., on one or more lateral sides of the introducer).

Therefore, in accordance with an important advantage of the present invention, the surgical access device can be used to guide an instrument along a specified path with respect to the main channel 622 so that it achieves accurate placement with respect to a specific location at the distal end of the access device.

FIGURE 22 illustrates another embodiment of a distal tip 708 of the present introducer. In this embodiment, the tip 708 is beveled so as to facilitate the insertion process; however, the extreme distal tip may also be rounded or blunt in order to avoid damage to internal tissue. The guide channel at the distal tip 708, which is shown folded back on the outer surface 735 of the split sheath 734, can be securely sealed to the body of the introducer by a heat seal process. Thus, the guide channel 520 is sealed and is impervious against contamination or distortion as the access device is introduced into the body. In another alternative embodiment of the distal tip, the distal end of the introducer may be shaped so as to have an annular mound at the distal tip of the guide channel and just proximal of the distal tip of the access device. Likewise, this mound can serve to protect the distal end of the guide channel against contamination or damage as the access device is introduced into the body and navigated through its various anatomy.

### Hysteroscopic or Biopsy Introducer

If the surgeon notices a cyst, polyp, foreign body, lesion, or other suspicious pathology, a therapeutic procedure or biopsy can be accomplished concurrently with the initial diagnostic procedure. Likewise, the present invention advantageously avoids pre-dilation to accommodate a larger diameter instrument in a planned, therapeutic procedure, such as "GIFT."

A wide variety of secondary instruments, such as biopsy graspers, scissors, lasers, electrodes, catheters, etc. can be introduced into the guide channel and used in accordance with the present invention. After choosing an appropriate instrument in light of the initial diagnostic procedure, the physician introduces the instrument into the secondary port 116. In reference to FIGURE 23, for instance, in the case of biopsying a potentially pathologic tissue specimen 774, graspers 713 can be used. After introducing the distal end or working portion 775 of the graspers 713 into the instrument port of the present device, the physician advances the graspers 713 through the guide channel 118. As the physician advances the secondary instrument through the guide channel, the guide channel 118 will expand no more than is necessary to accommodate the cross-sectional dimensions of the secondary instrument. Accordingly, dilation of the cervix is kept to a minimum, which minimizes patient discomfort.

The physician continues to advance the secondary instrument, such as the graspers 713, through the guide channel until the working portion of the secondary instrument extends through the distal end of the guide channel as illustrated in FIGURE 24. At this point, using the hysteroscope, the physician is able to simultaneously view the graspers 713 and the targeted tissue 774, thereby enabling the physician to further manipulate the secondary instrument to treat the target tissue, perform a biopsy, remove a foreign body, etc.

If a biopsy has been performed, the graspers 713 are withdrawn from the guide channel and the tissue specimen is sent to be examined by a pathologist. The graspers can then be reintroduced into the instrument portal and advanced through the guide channel to perform additional biopsies, if necessary.

After all biopsies have been performed, the hysteroscope and the access device are withdrawn from the patient. Because the guide channel provides minimal resistance to external forces without an instrument in place, the guide channel readily assumes a collapsed state in response to pressure exerted by surrounding tissue as the access device is withdrawn from the patient, thereby minimizing patient discomfort upon removal of the device.

In conclusion, the surgical access device of the present invention, including the guide channel and method for constructing same, represents a marked advancement in the art of secondary, expandable endoscopic surgical channels. Thus, it should be understood that the scope of the present invention is not to be limited by the illustrations or foregoing description thereof, but rather by the appended claims, and certain variations and modifications of this invention will suggest themselves to one of ordinary skill in the art.

## Claims

1. A surgical access device comprising:
an elongate tubular element (132) with an open distal end providing a first channel (122); and
at least one secondary channel (118) providing an auxiliary lumen for the insertion of an instrument, endoscope, or other visualisation device,
**characterized in that**
the secondary channel (118) being constructed from a substantially inelastic flexible membrane (140) having a pre-release storage position and a dilated position wherein the membrane (140) is dilated in use so as to allow the passage of the instrument, endoscope or other visualisation device.

2. The surgical device of claim 1, wherein the inelastic flexible membrane (140) is in the form of a flexible tubular sleeve having a central lumen extending axially there through,
the sleeve having a radially inward facing surface thereon and said sleeve having a greater outer diameter than that of the tubular element (132),
the sleeve being mounted on the tubular element (132) so as to be positioned along an outer surface of the tubular element (132), and
an elongate flexible tubular sheath (134) surrounding the elongate tubular element and having an axially extending lumen therein, and an axially extending slit in the wall thereof,
wherein the sheath (134) is eccentrically positioned about the sleeve, wherein the membrane is sandwiched between the tubular element (132) and the tubular sheath (134) and also extends out of the slit, thereby forming the secondary channel (118).

3. The surgical device of claims 1 or 2, wherein the inelastic flexible membrane (140) is substantially self-supporting when in the dilated position.

4. The surgical device of claim 2, wherein the elongate flexible tubular sheath (134) is formed from a durable, bio-compatible polymeric material.

5. The surgical device of claims 2 and 4, wherein the elongate flexible tubular sheath (134) is formed from nylon.

6. The surgical device of claim 1, wherein the inelastic flexible membrane (140) is a multi-lumen membrane (340) wherein the multiple lumens extend axially through the membrane (340), and
the first of the lumens is mounted on the tubular member (332) with the remaining one or more lumens forming the at least one secondary channel (324).

7. The surgical device of claims 1, 2 or 6, wherein the elongate tubular element (132) comprises stainless steel tubing.

8. The surgical device of claims 1, 2 or 6, wherein the secondary channel (118) comprising the inelastic flexible membrane (140) in the pre-release storage position is set in a self-retaining position closely conforming to an outer surface of the device.

9. The surgical device of claims 1, 2, 6 or 8, wherein when the secondary channel (118) is in the pre-release storage position, the inelastic flexible membrane (140) is folded to minimize the profile of the surgical device.

10. The surgical device of claims 1, 2, 6, 8 or 9, wherein the inelastic flexible membrane (140) of the secondary channel (118) is thermally, mechanically, and/or chemically settable so as to be self-retained in the pre-release storage position.

11. The surgical device of claims 1, 2 or 6, wherein the secondary channel (118) is capable of forming a wide variety of channel configurations when in the dilated position.

12. The surgical device of claims 6 and 7, wherein the inelastic flexible membrane (140) is secured to the tubular element (132) by adhesives.

13. The surgical device of claims 6 and 7, wherein the inelastic flexible membrane (140) is secured to the tubular element (132) by heat bonding.

14. The surgical device of claims 1, 2, 6-11, wherein the inelastic flexible membrane (140) ranges in thickness from 0.0127mm to 0.0508mm.

15. The surgical device of claim 1, further comprising an open-loop member (402) at a curved distal end of the first channel (404), the open-loop member having an aperture (408) formed in the distal end, the secondary channel (410) in fluid communication with the aperture formed through the open loop member (402).

## Patentansprüche

1. Chirurgische Zugriffsvorrichtung, die Folgendes umfasst:
ein längliches rohrförmiges Element (132) mit einem offenen distalen Ende, das einen ersten Kanal (122) bereitstellt; und
mindestens einen zweiten Kanal (118), der ein zusätzliches Lumen zum Einführen eines Instruments, Endoskops oder einer anderen Visualisierungsvorrichtung bereitstellt,
**dadurch gekennzeichnet, dass**
der zweite Kanal (118) aus einer im Wesentlichen unelastischen biegsamen Membran (140) aufgebaut ist, die eine der Freisetzung vorausgehende Lagerposition und eine dilatierte Position aufweist, in der die Membran (140) im Gebrauch dilatiert ist, um den Durchtritt des Instruments, Endoskops oder der anderen Visualisierungsvorrichtung zuzulassen.

2. Chirurgische Vorrichtung nach Anspruch 1, wobei die unelastische biegsame Membran (140) in Form einer biegsamen rohrförmigen Hülse mit einem zentralen Lumen ist, das sich axial durch diese erstreckt,
wobei die Hülse eine radial nach innen gewandte Fläche in sich aufweist, und die Hülse einen Außendurchmesser hat, der größer ist als derjenige des rohrförmigen Elements (132),
wobei die Hülse so am rohrförmigen Element (132) angebracht ist, dass sie entlang einer Außenfläche des rohrförmigen Elements (132) positioniert ist, und eine längliche, biegsame und rohrförmige Umhüllung (134) das längliche rohrförmige Element umgibt und in sich ein sich axial erstreckendes Lumen und einen sich in ihrer Wand axial erstreckenden Schlitz aufweist,
wobei die Umhüllung (134) exzentrisch um die Hülse positioniert ist, wobei die Membran zwischen dem rohrförmigen Element (132) und der rohrförmigen Umhüllung (134) eingeschoben ist und sich auch aus dem Schlitz heraus erstreckt, wodurch der zweite Kanal (118) gebildet ist.

3. Chirurgische Vorrichtung nach den Ansprüchen 1 oder 2, wobei die unelastische biegsame Membran (140), wenn sie sich in der dilatierten Position befindet, im Wesentlichen selbsttragend ist.

4. Chirurgische Vorrichtung nach Anspruch 2, wobei die längliche biegsame rohrförmige Umhüllung (134) aus einem dauerhaften, biokompatiblen Polymermaterial ausgebildet ist.

5. Chirurgische Vorrichtung nach den Ansprüchen 2 und 4, wobei die längliche biegsame rohrförmige Umhüllung (134) aus Nylon ausgebildet ist.

6. Chirurgische Vorrichtung nach Anspruch 1, wobei die unelastische biegsame Membran (140) eine Mehrlumenmembran (340) ist, wobei sich die mehreren Lumen axial durch die Membran (340) erstrecken, und
das erste der Lumen am rohrförmigen Teil (332) angebracht ist, wobei das übrige eine oder die übrigen mehreren Lumen den mindestens einen zweiten Kanal (324) bilden.

7. Chirurgische Vorrichtung nach den Ansprüchen 1, 2 oder 6, wobei das längliche rohrförmige Element (132) eine Rohrleitung aus rostfreiem Stahl umfasst.

8. Chirurgische Vorrichtung nach den Ansprüchen 1, 2 oder 6, wobei der zweite Kanal (118), der die unelastische biegsame Membran (140) in der der Freisetzung vorausgehenden Lagerposition umfasst, in eine selbstrückhaltende Position versetzt ist, die sich eng einer Außenfläche der Vorrichtung anpasst.

9. Chirurgische Vorrichtung nach den Ansprüchen 1, 2, 6 oder 8, wobei, wenn sich der zweite Kanal (118) in der der Freisetzung vorausgehenden Lagerposition befindet, die unelastische biegsame Membran (140) gefaltet ist, um das Profil der chirurgischen Vorrichtung zu minimieren.

10. Chirurgische Vorrichtung nach den Ansprüchen 1, 2, 6, 8 oder 9, wobei die unelastische biegsame Membran (140) des zweiten Kanals (118) wärmetechnisch, mechanisch und/oder chemisch fixierbar ist, um sich selbstrückhaltend in der der Freisetzung vorausgehenden Lagerposition zu befinden.

11. Chirurgische Vorrichtung nach den Ansprüchen 1, 2 oder 6, wobei der zweite Kanal (118) in der Lage ist, viele verschiedenartige Kanalgestaltungen zu bilden, wenn er sich in der dilatierten Position befindet.

12. Chirurgische Vorrichtung nach den Ansprüchen 6 und 7, wobei die unelastische biegsame Membran (140) durch Klebstoffe am rohrförmigen Element (132) befestigt ist.

13. Chirurgische Vorrichtung nach den Ansprüchen 6 und 7, wobei die unelastische biegsame Membran (140) durch Wärmebindung am rohrförmigen Element (132) befestigt ist.

14. Chirurgische Vorrichtung nach den Ansprüchen 1, 2, 6 bis 11, wobei die unelastische biegsame Membran (140) in der Dicke 0,0127 mm bis 0,0508 mm beträgt.

15. Chirurgische Vorrichtung nach Anspruch 1, darüber hinaus an einem gekrümmten distalen Ende des ersten Kanals (404) ein Teil (402) mit einem offenen Kreis umfassend, wobei das Teil mit dem offenen Kreis eine Öffnung (408) aufweist, die im distalen Ende ausgebildet ist, wobei der zweite Kanal (410) mit der Öffnung, die durch das Teil (402) mit dem offenen Kreis ausgebildet ist, in Fluidverbindung steht.

## Revendications

1. Dispositif d'accès chirurgical comprenant :
un élément tubulaire allongé (132) comportant une extrémité distale ouverte et constituant un premier canal (122) ; et
au moins un canal secondaire (118) constituant un lumen auxiliaire pour l'insertion d'un instrument, endoscope ou autre dispositif de visualisation,
**caractérisé en ce que**
le canal secondaire (118) est construit à partir d'une membrane flexible substantiellement non élastique (140) possédant une position de stockage de pré-relaxation et une position dilatée dans laquelle la membrane (140) est dilatée en utilisation de façon à autoriser le passage de l'instrument, endoscope ou autre dispositif de visualisation.

2. Dispositif chirurgical selon la revendication 1, dans lequel une membrane flexible non élastique (140) est sous la forme d'un manchon tubulaire flexible possédant un lumen central s'étendant en lui dans le sens axial,
le manchon possédant sur lui une surface dirigée dans le sens radial vers l'intérieur et ledit manchon possédant un diamètre extérieur supérieur à celui de l'élément tubulaire (132),
le manchon étant monté sur l'élément tubulaire (132) de façon à être positionné le long d'une surface extérieure de l'élément tubulaire (132), et
une gaine tubulaire flexible allongée (134) entourant l'élément tubulaire allongé et comportant à l'intérieur un lumen s'étendant dans le sens axial et une fente s'étendant dans le sens axial dans la paroi de celle-ci,
dans lequel la gaine (134) est positionnée de façon excentrique autour du manchon, dans lequel la membrane est prise en sandwich entre l'élément tubulaire (132) et la gaine tubulaire (134) et s'étend également hors de la fente, formant ainsi le canal secondaire (118).

3. Dispositif chirurgical selon les revendications 1 ou 2, dans lequel la membrane flexible non élastique (140) est substantiellement autoporteuse lorsqu'elle est en position dilatée.

4. Dispositif chirurgical selon la revendication 2, dans lequel la gaine tubulaire flexible allongée (134) est formée à partir d'un matériau polymère, biocompatible, durable.

5. Dispositif chirurgical selon les revendications 2 et 4, dans lequel la gaine tubulaire flexible allongée (134) est formée à partir de Nylon.

6. Dispositif chirurgical selon la revendication 1, dans lequel la membrane flexible non élastique (140) est une membrane multilumen (340) dans laquelle les multiples lumen s'étendent dans le sens axial au travers de la membrane (340), et
le premier des lumen est monté sur le membre tubulaire (332), les un ou plus lumens restants formant le au moins un canal secondaire (324).

7. Dispositif chirurgical selon l'une quelconque des revendications 1, 2 ou 6, dans lequel l'élément tubulaire allongé (132) comprend un tubage en acier inoxydable.

8. Dispositif chirurgical selon l'une quelconque des revendications 1, 2 ou 6, dans lequel le canal secondaire (118) comprenant la membrane flexible non élastique (140) en position de stockage de pré-relaxation est mis en une position d'auto-maintien se conformant de façon étroite à une surface extérieure du dispositif.

9. Dispositif chirurgical selon l'une quelconque des revendications 1, 2, 6 ou 8,
dans lequel lorsque le canal secondaire (118) est en position de stockage de pré-relaxation, la membrane flexible non élastique (140) est pliée pour minimiser le profil du dispositif chirurgical.

10. Dispositif chirurgical selon l'une quelconque des revendications 1, 2, 6, 8 ou 9,
dans lequel la membrane flexible non élastique (140) du canal secondaire (118) peut être réglée thermiquement, mécaniquement et/ou chimiquement de façon à être auto-maintenue dans la position de stockage de pré-relaxation.

11. Dispositif chirurgical selon l'une quelconque des revendications 1, 2 ou 6, dans lequel le canal secondaire (118) est capable de former une grande variété de configurations de canal lorsqu'il est en position dilatée.

12. Dispositif chirurgical selon les revendications 6 et 7, dans lequel la membrane flexible non élastique (140) est fixée à l'élément tubulaire (132) au moyen d'adhésifs.

13. Dispositif chirurgical selon les revendications 6 et 7, dans lequel la membrane flexible non élastique (140) est fixée à l'élément tubulaire (132) par scellement à chaud.

14. Dispositif chirurgical selon l'une quelconque des revendications 1, 2, 6 à 11,
dans lequel l'épaisseur de la membrane flexible non élastique (140) est dans la plage des 0,0127 mm à 0,0508 mm.

15. Dispositif chirurgical selon la revendication 1, comprenant en outre un organe en boucle ouverte (402) en une extrémité distale incurvée du premier canal (404), l'organe en boucle ouverte possédant une ouverture (408) formée à l'extrémité distale, le canal secondaire (410) étant en communication de fluide avec l'ouverture formée au travers de l'organe en boucle ouverte (402).
